# EUROPEAN PATENT SPECIFICATION

(11) **EP 1 978 994 B1**
(45) Date of publication and mention of the grant of the patent: **01.02.2012**
(21) Application number: 06849993.8
(22) Date of filing: 20.12.2006
(51) Int. Cl.: C07K 14/51, C07K 14/495, A61K 38/00, C12P 21/04

(54) **BMP-7 VARIANT COMPOSITIONS, METHODS AND USES**
BMP-7-VARIANTEN-ZUSAMMENSETZUNGEN, VERFAHREN UND VERWENDUNGEN
COMPOSITIONS À BASE DE VARIANTES DE BMP-7, PROCÉDÉS ET UTILISATIONS

(30) Priority: 22.12.2005 US 752969 P
(43) Date of publication of application: 15.10.2008
(73) Proprietor: Janssen Biotech, Inc., Horsham, PA 19044 (US)
(72) Inventor: AMEGADZIE, Bernard, Phoenixville, PA 19460 (US); CUNNINGHAM, Mark R., Kennett Square, PA 19348 (US); LUO, Jeffrey, Malvern, PA 19355 (US); MILLS, Juliane, Wallingford, PA 19086 (US); SWENCKI-UNDERWOOD, Bethany, Chester Springs, PA 19425 (US)
(74) Representative: Marshall, Cameron John
(86) International application number: PCT/US2006/048730
(87) International publication number: WO 2007/087053

(56) References cited:
- WO-A-00/20591
- WO-A-94/03600
- WO-A-99/03887
- WO-A-99/37320
- WO-A2-2005/097825
- US-A1- 2002 028 453
- US-A1- 2005 114 037
- US-A1- 2005 255 141
- DATABASE GENBANK [Online] 06 November 2005 GREGORY ET AL.: 'Bone morphogenetic protein 7 precursor [Homo sapiens]', XP008118198 Database accession no. (NP_001710)
- SWENCKI-UNDERWOOD ET AL: "Expression and characterization of a human BMP-7 variant with improved biochemical properties", PROTEIN EXPRESSION AND PURIFICATION, ACADEMIC PRESS, SAN DIEGO, CA, vol. 57, no. 2, 21 December 2007 (2007-12-21), pages 312-319, XP022398606, ISSN: 1046-5928, DOI: DOI:10.1016/J.PEP.2007.09.016

## Description

### Field of the Invention

The present invention relates to human Bone Morphogenic Protein 7 (hBMP-7) variant peptide chains, nucleic acid chains encoding these peptide chains, and methods of making and using the foregoing.

### Background of the Invention

Human Bone Morphogenic Protein 7 (hBMP-7) is a member of the TGF-β superfamily of proteins and has recognized therapeutic potential as both a modulator of bone structure and renal function (Klahr, J. Nephrol. 16:179-185 (2003)). For example, active hBMP-7 could be used as a protein therapeutic in indications such as myelofibrosis, idiopathic pulmonary fibrosis, renal osteodystrophy, diabetic nephropathy, chronic obstructive pulmonary disorder (COPD), and osteoarthritis.

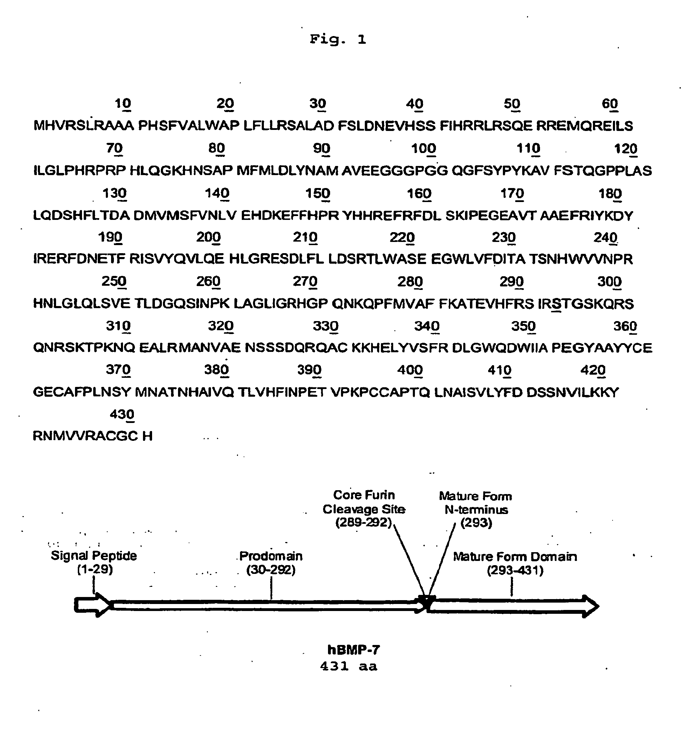

hBMP-7 is transcribed as mRNA containing a 1293 base pair open reading frame (the corresponding cDNA is shown at SEQ ID NO: 1) which is translated as a precursor protein of 431 amino acid residues (Fig 1; SEQ ID NO: 2) that is proteolytically processed. The hBMP-7 precursor protein contains a signal peptide spanning residues 1 to 29 of SEQ ID NO: 2, a prodomain spanning residues 30-292 of SEQ ID NO: 2, and a mature form domain spanning residues 293-431 of SEQ ID NO: 2 (Fig. 1).

Proteolytic processing of the hBMP-7 precursor protein is believed to occur in several steps. First, the signal peptide directs hBMP-7 to the endoplasmic reticulum of the cell where folding occurs and hBMP-7 forms a homodimeric protein complex. Next, the hBMP-7 prodomain is removed by proteolytic processing to produce a covalently linked homodimer consisting of two antiparallel hBMP-7 mature domain peptide chains. This final homodimeric protein complex is the biologically active form and secreted by cells.

Secreted hBMP-7 interacts with surface receptors an other cells and soluble antagonists, such as ActRII, BMPR1a, and Noggin to mediate its biological effects and activity. Structural information concerning these interactions can be derived from a number of different BMP-7 crystal structures (Greenwald et al., Mol. Cell. 11:605-017 (2003); Griffith et al., PNAS. 93:878-883 (1996); Keller et al., Nat. Struct. Mol. Biol. 5:481-488 (2004); Groppe et al., Nature. 420(6916):636-642 (2002).

Although hBMP-7 (formerly known as osteogenic protein 1 (OP-1) by Ozkaynak et al. (EMBO J. 9(7), 2085-2093 (1990)) has been known at least since 1990, to date several unresolved problems have prevented its development as a therapeutic protein. First, recombinant expression of hBMP-7 in mammalian cells is extremely low relative to other proteins of similar size. This is incompatible with the large-scale commercial production and purification needed for therapeutic use of hBMP-7. Second, proteolytic processing of the mature domain of hBMP-7 can occur at any of four different sites in that domain. This results in a number of different mature forms of hBMP-7 being produced during the recombinant expression of this protein. This high degree of heterogeneity in the expressed protein is highly undesirable in a protein therapeutic. Third, the mature form of hump-7 has poor solubility at pH values near 7.0; thus, acidic pH values are required to maintain hBMP-7 solubility. However, acidic pH values are incompatible with most common delivery methods, such as intravenous injection, used to administer protein therapeutics to patients. Lastly, the prodomain of hBMP-7 associates non-covalently with the mature domain of hBMP-7. Thus, purifying the prodomain away from the mature domain of hBMP-7 is relatively difficult and is incompatible with large-scale commercial production and purification.

Thus, a need exists for hBMP-7 variant peptide chains with improved properties that are suitable for use as therapeutic proteins and are compatible with large-scale commercial production and purification.

WO 99/37320 discloses methods and compositions for enhancing cognitive function using morphogenic proteins. US 2005114037 discloses methods for rational pegylation of proteins. WO 99/03887 discloses derivatives of growth hormone and related proteins. WO2005/097825 discloses BMP-7 variants.

### Brief Description of the Drawings

Fig. 1 shows the wild-type hBMP-7 precursor amino acid sequence and hBMP-7 amino acid sequence feature map. Underlined bold text denotes the amino terminus of mature hBMP-7.
Fig. 2 shows expression of hBMP-7 variants encoded by p3104, p3105, p3106, p3107, and p3108 as assayed by sandwich-type ELISA.

### Summary of the Invention

One aspect of the disclosure is an isolated peptide chain comprising the amino acid sequence shown in SEQ ID NO: 4.

Another aspect of the disclosure is an isolated peptide chain comprising the amino acid sequence shown in SEQ ID NO: 6.

Another aspect of the disclosure is an isolated peptide chain comprising the amino acid sequence shown in SEQ ID NO: 8.

Another aspect of the disclosure is an isolated peptide chain comprising the amino acid sequence shown in SEQ ID NO: 10.

Another aspect of the disclosure is an isolated peptide chain comprising the amino acid sequence shown in SEQ ID NO: 12.

Another aspect of the disclosure is an isolated peptide chain comprising the amino acid sequence shown in SEQ ID NO: 14.

Another aspect of the disclosure is an isolated peptide chain comprising the amino acid sequence shown in SEQ ID NO: 16.

Another aspect of the disclosure is an isolated peptide chain comprising the amino acid sequence shown in SEQ ID NO: 18.

Another aspect of the disclosure is an isolated peptide chain comprising the amino acid sequence shown in SEQ ID NO: 20.

Another aspect of the disclosure is an isolated peptide chain comprising the amino acid sequence shown in SEQ ID NO: 22.

Another aspect of the disclosure is an isolated peptide chain comprising the amino acid sequence shown in SEQ ID NO: 24.

Another aspect of the disclosure is an isolated peptide chain comprising the amino acid sequence shown in SEQ ID NO: 26.

Another aspect of the disclosure is an isolated peptide chain comprising the amino acid sequence shown in SEQ ID NO: 28.

Another aspect of the disclosure is an isolated peptide chain comprising the amino acid sequence shown in SEQ ID NO: 30.

Another aspect of the disclosure is an isolated peptide chain comprising the amino acid sequence shown in SEQ ID NO: 32.

Another aspect of the disclosure is an isolated peptide chain comprising the amino acid sequence shown in SEQ ID NO: 34.

Another aspect of the disclosure is an isolated peptide chain comprising the amino acid sequence shown in SEQ ID NO: 36.

Another aspect of the disclosure is an isolated peptide chain comprising the amino acid sequence shown in SEQ ID NO: 38.

Another aspect of the disclosure is an isolated peptide chain comprising the amino acid sequence shown in SEQ ID NO: 40.

The invention provides an isolated peptide chain comprising the amino acid sequence shown in SEQ ID NO: 42.

Another aspect of the disclosure is an isolated nucleic acid comprising the nucleic acid sequence shown in SEQ ID NO: 3.

Another aspect of the disclosure is an isolated nucleic acid comprising the nucleic acid sequence shown in SEQ ID NO: 5.

Another aspect of the disclosure is an isolated nucleic acid comprising the nucleic acid sequence shown in SEQ ID NO: 7.

Another aspect of the disclosure is an isolated nucleic acid comprising the nucleic acid sequence shown in SEQ ID NO: 9.

Another aspect of the disclosure is an isolated nucleic acid comprising the nucleic acid sequence shown in SEQ ID NO: 11.

Another aspect of the disclosure is an isolated nucleic acid comprising the nucleic acid sequence shown in SEQ ID NO: 13.

Another aspect of the disclosure is an isolated nucleic acid comprising the nucleic acid sequence shown in SEQ ID NO: 15.

Another aspect of the disclosure is an isolated nucleic acid comprising the nucleic acid sequence shown in SEQ ID NO: 17.

Another aspect of the disclosure is an isolated nucleic acid comprising the nucleic acid sequence shown in SEQ ID NO: 19.

Another aspect of the disclosure is an isolated nucleic acid comprising the nucleic acid sequence shown in SEQ ID NO: 21.

Another aspect of the disclosure is an isolated nucleic acid comprising the nucleic acid sequence shown in SEQ ID NO: 23.

Another aspect of the disclosure is an isolated nucleic acid comprising the nucleic acid sequence shown in SEQ ID NO: 25.

Another aspect of the disclosure is an isolated nucleic acid comprising the nucleic acid sequence shown in SEQ ID NO: 27.

Another aspect of the disclosure is an isolated nucleic acid comprising the nucleic acid sequence shown in SEQ ID NO: 29.

Another aspect of the disclosure is an isolated nucleic acid comprising the nucleic acid sequence shown in SEQ ID NO: 31.

Another aspect of the disclosure is an isolated nucleic acid comprising the nucleic acid sequence shown in SEQ ID NO: 33.

Another aspect of the disclosure invention is an isolated nucleic acid comprising the nucleic acid sequence shown in SEQ ID NO: 35.

Another aspect of the disclosure is an isolated nucleic acid comprising the nucleic acid sequence shown in SEQ ID NO: 37.

Another aspect of the disclosure is an isolated nucleic acid comprising the nucleic acid sequence shown in SEQ ID NO: 39.

The invention provides an isolated nucleic acid comprising the nucleic acid sequence shown in SEQ ID NO: 41.

### Detailed Description of the Invention

As used herein and in the claims, the singular forms "a," "and," and "the" include plural reference unless the context clearly dictates otherwise. Thus, for example, reference to "a cell" is a reference to one or more cells and includes equivalents thereof known to those skilled in the art.

Unless defined otherwise, all technical and scientific terms used herein have the same meaning as commonly understood by one of ordinary skill in the art to which this invention belongs. Although any compositions and methods similar or equivalent to those described herein can be used in the practice or testing of the invention, exemplary compositions and methods are described herein.

The term "peptide chain" means a molecule comprising at least two amino acid residues linked by a peptide bond to form a chain. Large peptide chains of more than 50 amino acids may be referred to as "polypeptides" or "proteins." Small peptide chains of less than 50 amino acids may be referred to as "peptides."

The term "nucleic acid" means a molecule comprising at least two nucleic acid residues linked to form a chain. Such nucleic acid residues may be those found in DNA or RNA. Small nucleic acids of less than 50 residues may be referred to as "oligonucleotides."

The term "hBMP-7 responsive condition" means a pathological condition that is responsive to a biological activity of a hBMP-7 peptide chain or a hBMP-7 variant peptide chain. Such peptide chains may comprise a fragment of SEQ ID NO: 2 or a peptide chain of the invention. Examples of hBMP-7 activity responsive pathological conditions include, for example, myelofibrosis, idiopathic pulmonary fibrosis, renal osteodystrophy, diabetic nephropathy, chronic obstructive pulmonary disorder (COPD), and osteoarthritis. Biological activities resulting from hBMP-7 peptide chain or a hBMP-7 variant peptide chain include, for example, induction of bone formation, increased of alkaline phosphatase activity, increased hyaluronan synthase 2 mRNA levels, increased type I collagen mRNA levels, increased osteocalcin synthesis, and inhibition of TGF-β induced cell proliferation. Those skilled in the art will recognize many other such biological activities and pathological conditions.

The invention provides isolated peptide chain hBMP-7 variant compositions, nucleic acids encoding these compositions, and related methods. The compositions and methods of the invention will be useful in controlling cell proliferation and in the treatment of hBMP-7 responsive conditions such as myelofibrosis, idiopathic pulmonary fibrosis, renal osteodystrophy, diabetic nephropathy, chronic obstructive pulmonary disorder (COPD), and osteoarthritis.

All peptide chains and nucleic acids of the invention were derived by mutagenesis using standard in vitro or in vivo methods, or combinations of such methods, from the cDNA (SEQ ID NO: 1) encoding the wild-type hBMP-7 precursor peptide chain of SEQ ID NO: 2. All mutated positions in the hBMP-7 variant peptide chains of the invention are described herein by referencing the position of the amino acid residue that has been changed in the wild-type hBMP-7 precursor peptide chain of SEQ ID NO: 2. Conventional one and three-letter amino acid codes are used herein as follows:

| Amino acid | Three-letter code | One-letter code |
|---|---|---|
| Alanine | ala | A |
| Arginine | arg | R |
| Asparagine | asn | N |
| Aspartate | asp | D |
| Cysteine | cys | C |
| Glutamate | glu | E |
| Glutamine | gln | Q |
| Glycine | gly | G |
| Histidine | his | H |
| Isoleucine | ile | I |
| Leucine | leu | L |
| Lysine | lys | K |
| Methionine | met | M |
| Phenylalanine | phe | F |
| Proline | pro | P |
| Serine | ser | S |
| Threonine | thr | T |
| Tryptophan | trp | W |
| Tyrosine | tyr | Y |
| Valine | val | V |

Single letter amino acid abbreviations are used to describe the nature of the change. For example, R299S denotes that the R (arginine) amino acid at position 299 of SEQ ID NO: 2 has been changed to an S (serine) amino acid and ΔS293-R299 indicates that the amino acid sequence starting at S amino acid at position 293 of SEQ ID NO: 2 and ending at the R at position 299 of SEQ ID NO: 2 has been deleted.

Wild-type hBMP-7 (SEQ ID NO: 2) has several important properties and features which may be modified by mutation. First, multiple mature hBMP-7 isoforms, each with different amino termini, can be produced during proteolytic processing of the hBMP-7 precursor protein (SEQ ID NO: 2). These termini may form at S293, R299, R314, and M315 of the wild-type hBMP-7 precursor protein (SEQ ID NO: 2). The 5293, R299S, R314S, ΔS293-R299, ΔS293-R314, and ΔS293-M315 mutations were each designed to prevent the formation of multiple mature hBMP-7 isoforms with different amino termini by eliminating cleavage in hBMP-7 variant peptide chains at the positions affected by each mutation. The H287R mutation flanks a core furin cleavage site located in the hBMP-7 prodomain of SEQ ID NO: 2. The H287R mutation was designed to enhance furin cleavage events in hBMP-7 variant peptide chains that produce an amino terminus at S293 of SEQ ID NO: 2.

Second, mature hBMP-7 forms complexes with the ActRII and BMPPR1a receptor proteins. The L407K, F409K, L417R, L382K, L382N, V383K, I386R, and I386N mutations were designed to decrease the surface hydrophobicity of hBMP-7 variant peptide chains while at the same time minimizing any disruption to hBMP-7 variant:ActRII or hBMP-7 variant:BMPPR1a peptide chain complex formation. Such decreased surface hydrophobicity in hBMP-7 variant peptide chains is designed to reduce the formation of aggregates by variants containing these mutations while still permitting hBMP-7 mediated signaling through the ActRII and BMPPR1a receptor proteins.

Third, mature hBMP-7 can be bound by the soluble antagonist protein Noggin. The W347D mutation was designed to decrease hBMP-7 variant peptide chain binding to Noggin to enhance hBMP-7 mediated signaling and biological activity.

Additionally, mutations at R421, N422, and R426 of hBMP-7 may increase expression of the resulting hBMP-7 variant peptide chain. Without wishing to be limited by any theory, it is believed that mutations such as R421E, N422D, and R426E improve hBMP-7 variant peptide chain expression by increasing the solubility of the expressed peptide chain.

Further, the pharmacological properties of hBMP-7 variant peptide chains, such as *in vivo* half-life, may be improved by pegylation. The S293C mutation is designed to create an amino terminal C residue in the mature form of the hBMP-7 variant peptide chains containing this mutation. This amino terminal C residue can then be post-translationally modified by the covalent attachment of polyethylene glycol by techniques known to those skilled in the art.

Last, deletion and domain replacement mutants derived from the wild-type hBMP-7 precursor peptide chain of SEQ ID NO: 2 can be designed to improve hBMP-7-variant peptide chain expression. One such hBMP-7 variant peptide chain mutant comprises the hBMP-7 signal peptide and prodomain alone, but lacks the hBMP-7 mature form domain which has been deleted. Another such mutant comprises the human growth hormone signal peptide (residues M1 to A26 of SEQ ID NO: 38) which replaces the native hBMP-7 secretory signal peptide, lacks the hBMP-7 prodomain which has been deleted, and contains the R299S and R314S mutations in the hBMP-7 mature form domain. Another mutant comprises the human BMP-2 (hBMP-2) secretory signal peptide instead of the native hBMP-7 secretory signal peptide, the hBMP-2 prodomain instead of the native hBMP-7 prodomain, and the R299S and R314S mutations in the hBMP-7 mature form domain. These hBMP-7 variant peptide chain mutants were designed to facilitate improved secretion, proteolytic processing and expression of hBMP-7.

Each individual mutation described herein, such as point mutations, deletions, or domain replacements can be used alone or in combination to produce the peptide chains of the invention. The mutations described here may also be combined to generate additional variant BMP-7 peptide chains having modified properties corresponding, in part, to the various individual mutations described herein. Nucleic acids encoding such additional variant peptide chains may be generated using standard *in vitro* or *in vivo* methods, or combinations of such methods, well known in the art. The peptide chains of the invention may also be poat-translationally modified by the addition of one or more covalent modifications such as, for example, the addition of polyethylene glycol to the amino terminus or other portions of the peptide chains of the invention. Lastly, the signal peptide and prodomain sequences of the variant peptides of the invention may be replaced with functionally equivalent amino acid sequences. Such additional variants and the nucleic acids encoding them are also within the scope of the invention. Similarly, variant peptide chains comprising mutated mature domain peptide chains alone and the nucleic acids encoding them are also within the scope of the invention.

One aspect of the disclosure is an isolated peptide chain comprising the amino acid sequence shown in SEQ ID NO: 4. The peptide chain of SEQ ID NO: 4 was derived from the wild-type hBMP-7 precursor peptide chain of SEQ ID NO: 2 and contains the R299S the R314S mutations.

Another aspect of the disclosure is an isolated peptide chain comprising the amino acid sequence shown in SEQ ID NO: 6. The peptide chain of SEQ ID NO: 6 was derived from the wild-type hBMP-7 precursor peptide chain of SEQ ID NO: 2 and contains the ΔS293-R299, and R314S mutations.

Another aspect of the disclosure is an isolated peptide chain comprising the amino acid sequence shown in SEQ ID NO: 8. The peptide chain of SEQ ID NO: 8 was derived from the wild-type hBMP-7 precursor peptide chain of SEQ ID NO: 2 and contains the ΔS293-M314 mutation.

Another aspect of the disclosure is an isolated peptide chain comprising the amino acid sequence shown in SEQ ID NO: 10. The peptide chain of SEQ ID NO: 10 was derived from the wild-type hBMP-7 precursor peptide chain of SEQ ID NO: 2 and contains the ΔS293-M325 mutation.

Another aspect of the disclosure is an isolated peptide chain comprising the amino acid sequence shown in SEQ ID NO: 12. The peptide chain of SEQ ID NO: 12 was derived from the wild-type hBMP-7 precursor peptide chain of SEQ ID NO: 2 and contains the R299S, R314S, and L407K mutations.

Another aspect of the disclosure is an isolated peptide chain comprising the amino acid sequence shown in SEQ ID NO: 14. The peptide chain of SEQ ID NO: 14 was derived from the wild-type hBMP-7 precursor peptide chain of SEQ ID NO: 2 and contains the R299S, R314S, and F409K mutations.

Another aspect of the disclosure is an isolated peptide chain comprising the amino acid sequence shown in SEQ ID NO: 16. The peptide chain of SEQ ID NO: 16 was derived from the wild-type hBMP-7 precursor peptide chain of SEQ ID NO: 2 and contains the R2999, R314S, and L417R mutations.

Another aspect of the disclosure is an isolated peptide chain comprising the amino acid sequence shown in SEQ ID NO: 18. The peptide chain of SEQ ID NO: 18 was derived from the wild-type hBMP-7 precursor peptide chain of SEQ ID NO: 2 and contains the R299S, R3148, and L382K mutations.

Another aspect of the disclosure is an isolated peptide chain comprising the amino acid sequence shown in SEQ ID NO: 20. The peptide chain of SEQ ID NO: 20 was derived from the wild-type hBMP-7 precursor peptide chain of SEQ ID NO: 2 and contains the R299S, R314S, and L382N mutations.

Another aspect of the disclosure is an isolated peptide chain comprising the amino acid sequence shown in SEQ ID NO: 22. The peptide chain of SEQ in NO: 22 was derived from the wild-type hBMP-7 precursor peptide chain of SEQ ID NO: 2 and contains the R299S, R324S, and V383K mutations.

Another aspect of the disclosure is an isolated peptide chain comprising the amino acid sequence shown in SEQ ID NO: 24. The peptide chain of SEQ ID NO: 24 was derived from the wild-type hBMP-7 precursor peptide chain of SEQ ID NO: 2 and contains the R299S, R314S, and I386R mutations.

Another aspect of the disclosure is an isolated peptide chain comprising the amino acid sequence shown in SEQ ID NO: 26. The peptide chain of SEQ ID NO: 26 was derived from the wild-type hBMP-7 precursor peptide chain of SEQ ID NO: 2 and contains the R299S, R314S, and I386N mutations.

Another aspect of the disclosure is an isolated peptide chain comprising the amino acid sequence shown in SEQ ID NO: 28. The peptide chain of SEQ ID NO: 28 was derived from the wild-type hBMP-7 precursor peptide chain of SEQ ID NO: 2 and contains the R299S, R314S, V383K, L407K, and F409K mutations.

Another aspect of the disclosure is an isolated peptide chain comprising the amino acid sequence shown in SEQ ID NO: 30. The peptide chain of SEQ ID NO: 30 was derived from the wild-type hBMP-7 precursor peptide chain of SEQ ID NO: 2 and contains the R299S, R314S, and W347D mutations.

Another aspect of the disclosure is an isolated peptide chain comprising the amino acid sequence shown in SEQ ID NO: 32. The peptide chain of SEQ ID NO: 32 was derived from the wild-type hBMP-7 precursor peptide chain of SEQ ID NO: 2 and contains the R299S, R314S, R421E, N422D and R426E mutations.

Another aspect of the disclosure is an isolated peptide chain comprising the amino acid sequence shown in SEQ ID NO: 34. The peptide chain of SEQ ID NO: 34 was derived from the wild-type hBMP-7 precursor peptide chain of SEQ ID NO: 2 and contains the S293C, R299S. and R314S mutations.

In one embodiment the disclosure provides an isolated peptide chain comprising the amino acid sequence shown in SEQ ID NO: 34 further comprising at least one polyethylene glycol molecule. Such a polyethylene glycol residue may be covalently linked to C293 of SEQ ID NO: 34 for example. C293 is expected to be the amino terminal residue in the mature form of SEQ ID NO: 34 resulting from proteolytic processing.

Another aspect of the disclosure is an isolated peptide chain comprising the amino acid sequence shown in SEQ ID NO: 36. The peptide chain of SEQ ID NO: 36 was derived from the wild-type hBMP-7 precursor peptide chain of SEQ ID NO: 2. The peptide chain of SEQ ID NO: 36 comprises the hBMP-7 signal peptide (residues M1 to A29 of SEQ ID NO: 2) and prodomain alone (residues D30 to R292 of SEQ ID NO: 20; the hBMP-7 mature form domain (residues S293 to H431 of SEQ ID NO: 2) has been deleted.

Another aspect of the disclosure is an isolated peptide chain comprising the amino acid sequence shown in SEQ ID NO: 38. The peptide chain of SEQ ID NO: 38 was derived, in part, from the wild-type hBMP-7 precursor peptide chain of SEQ ID NO: 2. The peptide chain of SEQ ID NO: 38 comprises a human growth hormone signal peptide (residues M1 to A26 of SEQ ID NO: 38), lacks the hBMP-7 prodomain, and contains the R299S and R314S mutations in the hBMP-7 mature form domain.

Another aspect of the disclosure is an isolated peptide chain comprising the amino acid sequence shown in SEQ ID NO: 40. The peptide chain of SEQ ID NO: 40 was derived, in part, from the wild-type hBMP-7 precursor peptide chain of SEQ ID NO: 2. The peptide chain of SEQ ID NO: 40 comprises the hBMP-2 secretory signal peptide instead of the native hBMP-7 secretory signal peptide, hBMP-2 prodomain instead of the native hBMP-7 prodomain, and the R299S and R314S mutations in the hBMP-7 mature form domain.

One aspect of the invention is an isolated peptide chain comprising the amino acid sequence shown in SEQ ID NO: 42. The peptide chain of SEQ ID NO: 42 was derived from the wild-type hBMP-7 precursor peptide chain of SEQ ID NO: 2. The peptide chain of SEQ ID NO: 42 contains the H287, R299S, R314S, R421E, N422D, and R4268 mutations.

One embodiment of the invention is an isolated nucleic acid comprising a nucleic acid sequence encoding a peptide chain of the invention. Such nucleic acids may be generated using standard in vitro or in vivo methods, or combinations of such methods, well known in the art. Such nucleic acids may comprise alternative codons encoding the amino acids of the peptide chains of the invention or organism optimized codons encoding the amino acids of the peptide chains of the invention.

Another aspect of the disclosure is an isolated nucleic acid comprising the nucleic acid sequence shown in SEQ ID NO: 3 (p3105; R299S/R314S).

Another aspect of the disclosure is an isolated nucleic acid comprising the nucleic acid sequence shown in SEQ ID NO: 5.

Another aspect of the disclosure is an isolated nucleic acid comprising the nucleic acid sequence shown in SEQ ID NO: 7.

Another aspect of the disclosure is an isolated nucleic acid comprising the nucleic acid sequence shown in SEQ ID NO: 9.

Another aspect of the disclosure is an isolated nucleic acid comprising the nucleic acid sequence shown in SEQ ID NO: 11.

Another aspect of the disclosure is an isolated nucleic acid comprising the nucleic acid sequence shown in SEQ ID NO: 13.

Another aspect, of the disclosure is an isolated nucleic acid comprising the nucleic acid sequence shown in SEQ ID NO: 15.

Another aspect of the disclosure is an isolated nucleic acid comprising the nucleic acid sequence shown in SEQ ID NO: 17.

Another aspect of the disclosure is an isolated nucleic acid comprising the nucleic acid sequence shown in SEQ ID NO: 19.

Another aspect of the disclosure is an isolated nucleic acid comprising the nucleic acid sequence shown in SEQ ID NO: 21.

Another aspect of the disclosure is an isolated nucleic acid comprising the nucleic acid sequence shown in SEQ ID NO: 23.

Another aspect of the disclosure is an isolated nucleic acid comprising the nucleic acid sequence shown in SEQ ID NO: 25.

Another aspect of the disclosure is an isolated nucleic acid comprising the nucleic acid sequence shown in SEQ ID NO: 27.

Another aspect of the disclosure is an isolated nucleic acid comprising the nucleic acid sequence shown in SEQ ID NO: 29.

Another aspect of the disclosure is an isolated nucleic acid comprising the nucleic acid sequence shown in SEQ ID NO: 31.

Another aspect of the disclosure is an isolated nucleic acid comprising the nucleic acid sequence shown in SEQ ID NO: 33.

Another aspect of the disclosure is an isolated nucleic acid comprising the nucleic acid sequence shown in SEQ ID NO: 35.

Another aspect of the disclosure is an isolated nucleic acid comprising the nucleic acid sequence shown in SEQ ID NO: 37.

Another aspect of the disclosure is an isolated nucleic acid comprising the nucleic acid sequence shown in SEQ ID NO: 39.

One aspect of the invention is an isolated nucleic acid comprising the nucleic acid sequence shown in SEQ ID NO: 41.

Another embodiment of the invention is an in vitro method of inhibiting TGF-β induced cell proliferation comprising providing a peptide chain of the invention to a cell. The peptide chains of the invention may be provided to cells that are, for example, eukaryotic cells. The peptide chains of the invention can be administered by any technique that provides such molecules to a cell. A peptide chain can be provided to a cell in vitro by, for example, supplementing the culture medium with the peptide chain or a nucleic acid chain encoding the peptide chain. Peptide chains may also be provided to a cell by transfecting a nucleic acid chain encoding the peptide chain into the cell. In one aspect of the disclosure a peptide chain may be provided to a cell in vivo by, for example, intravenous injection of the peptide chain or a nucleic acid encoding the peptide chain into an animal or tissue. Those skilled in the art will recognize other means for administering the peptide chains of the invention to a cell in vi tro or in vivo. Such means also include those modes for delivery of an agent to a host that are discussed below.

Another embodiment of the invention is the peptide chain of the invention for use in a method of treating myelofibrosis, idiopathic pulmonary fibrosis, renal osteodystrophy, diabetic nephropathy, chronic obstructive pulmonary disorder (CODP) or osteoarthritis.

The methods of treating as described above the may be used to treat an animal patient belonging to any classification. Examples of such animals include mammals such as humans, rodents, dogs, cats and farm animals and other animal classes such as birds, reptiles and fish.

The mode of administration for therapeutic use of the peptide chains of the invention may be any suitable route that delivers the peptide chain to the host. The peptide chains and nucleic acid chains of the invention are particularly useful for parenteral administration, i.e., subcutaneously, intramuscularly, intradermally, intravenously or intranasally.

Peptide chains and nucleic acids of the invention may be prepared as pharmaceutical compositions containing an effective amount of the peptide chain or nucleic acid as an active ingredient in a pharmaceutically acceptable carrier. An aqueous suspension or solution containing the peptide chain or nucleic acid, preferably buffered at physiological pH, in a form ready for injection is preferred. The compositions for parenteral administration will commonly comprise a solution of the antagonist of the invention or a cocktail thereof dissolved in an pharmaceutically acceptable carrier, preferably an aqueous carrier. A variety of aqueous carriers may be employed, e.g., 0.4% saline, 0.3% glycine and the like. These solutions are sterile and generally free of particulate matter. These solutions may be sterilized by conventional, well-known sterilization techniques (e.g., filtration). The compositions may contain pharmaceutically acceptable auxiliary substances as required to approximate physiological conditions such as pH adjusting and buffering agents, etc. The concentration of the peptide chains or nucleic acid chains of the invention in such pharmaceutical formulation can vary widely, i.e., from less than about 0.5%, usually at or at least about 1% to as much as 15 or 20% by weight and will be selected primarily based on fluid volumes, viscosities, etc., according to the particular mode of administration selected.

Thus, a pharmaceutical composition for intramuscular injection could be prepared to contain 1 mL sterile buffered water, and between about 1 ng to about 100 mg, e.g. about 50 ng to about 30 mg or more preferably, about 5 mg to about 25 mg, of a peptide chain or nucleic acid of the invention. Similarly, a pharmaceutical composition of the invention for intravenous infusion could be made up to contain about 250 ml of sterile Ringer's solution, and about 1 mg to about 30 mg and preferably 5 mg to about 25 mg of an antagonist of the invention. Actual methods for preparing parenterally administrable compositions are well known and are described in more detail in, for example, "Remington's Pharmaceutical Science", 15th ed., Mack Publishing Company, Easton, PA.

The peptide chains and nucleic acids of the invention, when in a pharmaceutical preparation, can be present in unit dose forms. The appropriate therapeutically effective dose can be determined readily by those of skill in the art. A determined dose may, if necessary, be repeated at appropriate time intervals selected as appropriate by a physician during the treatment period.

The peptide chains or nucleic acids of the invention can be lyophilized for storage and reconstituted in a suitable carrier prior to use. This technique has been shown to be effective with conventional protein and immunoglobulin preparations and art-known lyophilization and reconstitution techniques can be employed.

Another embodiment of the invention is a vector comprising at least one nucleic acid of the invention. Such vectors permit a nucleic acid of the invention to be introduced into a cell, animal or other nucleic acid replication system and expression systems, such as an in vitro transcription and translation system. For example a vector of the invention may comprise portions of a transposon, plasmid, virus, or chromosome that, alone or when combined, permit a nucleic acid of the invention to be introduced into a cell or other system. A wide variety of such vectors are well known to those skilled in the art.

Another embodiment of the invention is a host cell comprising a vector of the invention. Such host cells may be eukaryotic cells, bacterial cells, plant cells or archeal cells. Exemplary eukaryotic cells may be of mammalian, insect, avian or other animal origins. An exemplary mammalian eukaryotic cell line is the HEK-293 (American Type Culture Collection, Manassas, VA; ATCC^{®} Number: CRL-1573™) cell line. Other useful cell lines include those derived from Chinese Hamster Ovary (CHO) cells such as CHO-K1 (ATCC^{®} Number: CRL-61™) and rat osteosarcoma (ROS) cells such as ROS 27/2.8 cells. Mammalian eukaryotic cells also include immortalized cell lines such as hybridomas or myeloma cell lines such as SP2/0 (ATCC^{®} Number: CRL-1581™), NSO (European Collection of Cell Cultures (ECACC), Salisbury, Wiltshire, UK, ECACC No. 85110503), FO (ATCC^{®} Number: CRL-1646™) and Ag653 (ATCC^{®} Number: CRL-1580™) murine cell lines. An exemplary human myeloma cell line is U266 (ATCC^{®} Number: CRL-TIB-196™).

Another embodiment of the invention is a method of making a peptide chain comprising culturing a host cell of the invention and recovering the peptide chain produced by the host cell. Standard cell culture and enzymology methods well known in the art may be used to culture a host cell of the invention and to recover peptide chains produced by such host cells.

Another embodiment of the disclosure is a method of making a peptide chain comprising co-expressing the peptide chains of SEQ ID NO: 36 and SEQ ID NO: 38 in an expression system and recovering a peptide chain capable of increasing alkaline phosphatase activity in ROS 17/2.8 cells. Expression systems useful in the method of the invention may be, for example, an in vitro coupled transcription and translation system, or a cell or other expression system, such as an animal or plant.

hBMP-7 mature form activity can be determined by assaying for increased alkaline phosphatase activity in rat osteosarcoma 17/2.8 (ROS 17/2.8) cells treated with the mature form of hBMP-7 relative to untreated control ROS 17/2.8 cells. A peptide chain capable of increasing alkaline phosphatase activity in ROS 17/2.8 cells has hBMP-7 activity. Alkaline phosphatase based hBMP-7 assays using ROS 17/2.8 cells can be performed using standard cell culture and enzymology methods well known in the art such as, for example, those described by Maliakal et al., Growth Factors. 112(3):227-234 (1994). Those skilled in the art will also recognize that such assays for hBMP-7 activity may also be used to confirm the biological activity and effect of the compositions and methods of the invention.

The present invention will now be described with reference to the following specific, non-limiting examples.

### Example 1

### Expression of hBMP-7 Variants in Mammalian Cells Transiently Transfected with Plasmid DNAs

hBMP-7 variant proteins were recombinantly expressed in mammalian HEK-293 cells transiently transfected with plasmid DNAs encoding hexahistidine tagged forms of each variant (Fig. 1). HEK-293 cells (ATCC^{®} Number: CRL-1573™) were propagated using standard culture conditions and methods. Cells were transiently transfected with p3105, p3106, p3107 and p3108 using standard transfection methods. HEK-293 cells were also transiently transfected with p3104 as a control.

The p3104 control vector contains a cDNA (SEQ ID NO: 1) encoding the wild-type hBMP-7 precursor protein (SEQ ID NO: 2). The p3105 vector contains a cDNA (SEQ ID NO: 3) encoding the R299S/R314S hBMP-7 variant protein (SEQ ID NO: 4). The p3106 vector contains a cDNA (SEQ ID NO: 5) encoding the ΔS293-R299/R314S hBMP-7 variant protein (SEQ ID NO: 6). The p3107 vector contains a cDNA (SEQ ID NO: 7) encoding the ΔS293-R314 hBMP-7 variant protein (SEQ ID NO: 8). The p3108 vector contains a cDNA (SEQ ID NO: 9) encoding the ΔS293-M315 hBMP-7 variant protein (SEQ ID NO: 10). In each of these vectors the wild-type or variant hBMP-7 cDNA is fused in frame at its 3' end to a cDNA encoding a hexahistidine tag. Consequently, the proteins expressed by these vectors are all hexahistidine tagged at their carboxy termini.

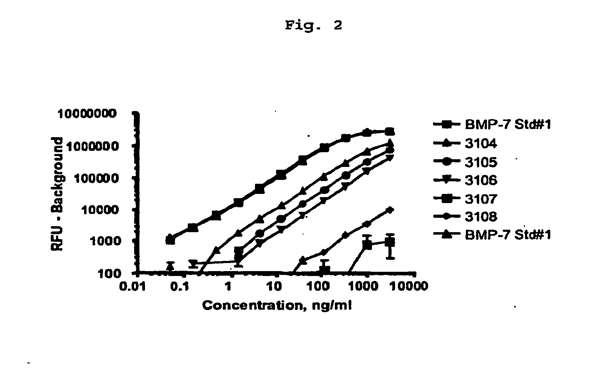

Cell culture supernatant was collected after transfection and hexahistidine tagged proteins were purified from the supernatant using Talon™ immobilized metal affinity chromatography resin (Clontech, Inc., Mountain View, CA). Hexahistidine purification using Talon™ resin and preparation of supernatants was performed as directed by the manufacturer. A standard sandwich-type ELISA specific for hBMP-7 was then performed on the resulting samples (Fig. 2). This type of ELISA can be used to confirm hBMP-7 variant expression by any cell type (e.g. CHO-K1). Positive control ELISAs were also performed on purified, wild-type, hBMP-7 samples. Assay data is expressed in Relative Light Units (RLU) minus background light emission (Fig. 2; Y-axis) versus the concentration of Talon™ resin purified protein (Fig. 2; x-axis).

The ELISA data indicates that all hBMP-7 variants were expressed in mammalian HEK-293 cells from the transiently transfected vector DNAs. Importantly, N-terminal sequencing analysis using standard methods demonstrated that S293 of SEQ ID NO: 2 is the amino terminal cleavage site necessary for formation of mature form hBMP-7.

## Claims

1. An isolated peptide chain comprising the amino acid sequence shown in SEQ ID NO: 42.

2. An isolated nucleic acid comprising a nucleic acid encoding the peptide chain of claim 1.

3. An isolated nucleic acid according to claim 2 comprising the nucleic acid sequence shown in SEQ ID NO: 41.

4. An *in vitro* method of inhibiting TGF-β induced cell proliferation comprising providing the peptide chain of claim 1 to a cell.

5. A vector comprising the nucleic acid of claim 2 or 3.

6. A host cell comprising the vector of claim 5.

7. A method of making a peptide chain comprising culturing the host cell of claim 6 and recovering the peptide chain produced by the host cell.

8. The peptide chain of claim 1 for use in a method of treating myelofibrosis, idiopathic pulmonary fibrosis, renal osteodystrophy, diabetic nephropathy, chronic obstructive pulmonary disorder (COPD), or osteoarthritis.

## Patentansprüche

1. Isolierte Peptidkette, umfassend die in SEQ ID Nr: 42 gezeigte Aminosäuresequenz.

2. Isolierte Nukleinsäure, umfassend eine die Peptidkette nach Anspruch 1 codierende Nukleinsäure.

3. Isolierte Nukleinsäure nach Anspruch 2, umfassend die in SEQ ID Nr: 41 gezeigte Nukleinsäuresequenz.

4. *In-vitro*-Verfahren zum Inhibieren der durch TGF-β induzierten Zellproliferation, bei dem man einer Zelle die Peptidkette nach Anspruch 1 zur Verfügung stellt.

5. Vektor, umfassend die Nukleinsäure nach Anspruch 2 oder 3.

6. Wirtszelle, umfassend den Vektor nach Anspruch 5.

7. Verfahren zur Herstellung einer Peptidkette, bei dem man die Wirtszelle nach Anspruch 6 kultiviert und die von der Wirtszelle produzierte Peptidkette isoliert.

8. Peptidkette nach Anspruch 1 zur Verwendung bei einem Verfahren zur Behandlung von Myelofibrose, idiopathischer Lungenfibrose, renaler Osteodystrophie, diabetischer Neuropathie, chronischer obstruktiver Atemwegserkrankung (Chronic Obstructive Pulmonary Disorder, COPD) oder Osteoarthritis.

## Revendications

1. Chaîne peptidique isolée, comprenant la séquence d'acides aminés présentée dans SEQ ID N°42.

2. Acide nucléique isolé comprenant un acide nucléique codant pour la chaîne peptidique de la revendication 1.

3. Acide nucléique isolé selon la revendication 2, comprenant la séquence d'acide nucléique présentée dans SEQ ID N°41.

4. Procédé *in vitro* d'inhibition de la prolifération cellulaire induite par le TGF-β, comprenant la fourniture, à une cellule, de la chaîne peptidique de la revendication 1.

5. Vecteur comprenant l'acide nucléique de la revendication 2 ou 3.

6. Cellule hôte comprenant le vecteur de la revendication 5.

7. Procédé de fabrication d'une chaîne peptidique, comprenant la culture de la cellule hôte de la revendication 6 et la récupération de la chaîne peptidique produite par la cellule hôte.

8. Chaîne peptidique de la revendication 1, pour utilisation dans un procédé de traitement de la myélofibrose, de la fibrose pulmonaire idiopathique, de l'ostéodystrophie rénale, de la néphropathie diabétique, de la brochopneumopathie chronique obstructive (BPCO) ou de l'ostéoarthrite.


### SEQUENCE LISTING

<110> AMEGADZIE, BERNARD
   CUNNINGHAM, MARK R. LUO, JINQUAN
   MILLS, JULIANE
   SWENCKI-UNDERWOOD, BETHANY
<120> BMP-7 VARIANT COMPOSITIONS, METHODS AND USES
<130> CEN5125PCT
<140> TO BE ASSIGNED
   <141> 2006-12-20
<150> 60/752,969
   <151> 2005-12-22
<160> 42
<170> FastSEQ for Windows Version 4.0
<210> 1
   <211> 1293
   <212> DNA
   <213> Homo sapiens
<400> 1
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggtcca cggggagcaa acagcgcagc 900
   cagaaccgct ccaagacgcc caagaaccag gaagccctgc ggatggccaa cgtggcagag 960
   aacagcagca gcgaccagag gcaggcctgt aagaagcacg agctgtatgt cagcttccga 1020
   gacctgggct ggcaggactg gatcatcgcg cctgaaggct acgccgccta ctactgtgag 1080
   ggggagtgtg ccttccctct gaactcctac atgaacgcca ccaaccacgc catcgtgcag 1140
   acgctggtcc acttcatcaa cccggaaacg gtgcccaagc cctgctgtgc gcccacgcag 1200
   ctcaatgcca tctccgtcct ctacttcgat gacagctcca acgtcatcct gaagaaatac 1260
   agaaacatgg tggtccgggc ctgtggctgc cac                               1293
<210> 2
   <211> 431
   <212> PRT
   <213> Homo sapiens
<400> 2
   Met His Val Arg Ser Leu Arg Ala Ala Ala Pro His Ser Phe Val Ala
   1               5                   10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Arg Ser Gln Asn Arg Ser
       290                 295                 300
   Lys Thr Pro Lys Asn Gln Glu Ala Leu Arg Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Val His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Leu Tyr Phe Asp Asp Ser Ser Asn Val Ile
                   405                 410                 415
   Leu Lys Lys Tyr Arg Asn Met Val Val Arg Ala Cys Gly Cys His
               420                 425                 430
<210> 3
   <211> 1293
   <212> DNA
   <213> Artificial Sequence
<220>
   <223> cDNA encoding a hBMP-7 precursor derived variant peptide chain comprising the R299S and R314S mutations.
<400> 3
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggtcca cggggagcaa acagagcagc 900
   cagaaccgct ccaagacgcc caagaaccag gaagccctga gcatggccaa cgtggcagag 960
   aacagcagca gcgaccagag gcaggcctgt aagaagcacg agctgtatgt cagcttccga 1020
   gacctgggct ggcaggactg gatcatcgcg cctgaaggct acgccgccta ctactgtgag 1080
   ggggagtgtg ccttccctct gaactcctac atgaacgcca ccaaccacgc catcgtgcag 1140
   acgctggtcc acttcatcaa cccggaaacg gtgcccaagc cctgctgtgc gcccacgcag 1200
   ctcaatgcca tctccgtcct ctacttcgat gacagctcca acgtcatcct gaagaaatac 1260
   agaaacatgg tggtccgggc ctgtggctgc cac                              1293
<210> 4
   <211> 431
   <212> PRT
   <213> Artificial Sequence
<220>
   <223> cDNA encoding a hBMP-7 precursor derived variant peptide chain comprising the R299S and R314S mutations.
<400> 4
   Met His Val Arg Ser Leu Arg Ala Ala Ala Pro His Ser Phe Val Ala
   1               5                   10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Ser Ser Gln Asn Arg Ser
       290                 295                 300
   Lys Thr Pro Lys Asn Gln Glu Ala Leu Ser Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Val His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Leu Tyr Phe Asp Asp Ser Ser Asn Val Ile
                   405                 410                 415
   Leu Lys Lys Tyr Arg Asn Met Val Val Arg Ala Cys Gly Cys His
               420                 425                 430
<210> 5
   <211> 1337
   <212> DNA
   <213> Artificial Sequence
<220>
   <223> cDNA encoding a hBMP-7 precursor derived variant peptide chain comprising the delta S293-R299 and R314S mutations.
<400> 5
   atgcacgtgc gctcactgcg agctgcggcg ccgcacagct tcgtggcgct ctgggcaccc 60
   ctgttatgca cgtgcgctca ctgcgagctg cggcgccgca cagcttcgtg gcgctctggg 120
   cacccctgtt cctgctgcgc tccgccctgg ccgacttcag cctggacaac gaggtgcact 180
   cgagcttcat ccaccggcgc ctccgcagcc aggagcggcg ggagatgcag cgcgagatcc 240
   tctccatttt gggcttgccc caccgcccgc gcccgcacct ccagggcaag cacaactcgg 300
   cacccatgtt catgctggac ctgtacaacg ccatggcggt ggaggagggc ggcgggcccg 360
   gcggccaggg cttctcctac ccctacaagg ccgtcttcag tacccagggc ccccctctgg 420
   ccagcctgca agatagccat ttcctcaccg acgccgacat ggtcatgagc ttcgtcaacc 480
   tcgtggaaca tgacaaggaa ttcttccacc cacgctacca ccatcgagag ttccggtttg 540
   atctttccaa gatcccagaa ggggaagctg tcacggcagc cgaattccgg atctacaagg 600
   actacatccg ggaacgcttc gacaatgaga cgttccggat cagcgtttat caggtgctcc 660
   aggagcactt gggcagggaa tcggatctct tcctgctcga cagccgtacc ctctgggcct 720
   cggaggaggg ctggctggtg tttgacatca cagccaccag caaccactgg gtggtcaatc 780
   cgcggcacaa cctgggcctg cagctctcgg tggagacgct ggatgggcag agcatcaacc 840
   ccaagttggc gggcctgatt gggcggcacg ggccccagaa caagcagccc ttcatggtgg 900
   ctttcttcaa ggccacggag gtccacttcc gcagcatccg gagccagaac cgctccaaga 960
   cgcccaagaa ccaggaagcc ctgagcatgg ccaacgtggc agagaacagc agcagcgacc 1020
   agaggcaggc ctgtaagaag cacgagctgt atgtcagctt ccgagacctg ggctggcagg 1080
   actggatcat cgcgcctgaa ggctacgccg cctactactg tgagggggag tgtgccttcc 1140
   ctctgaactc ctacatgaac gccaccaacc acgccatcgt gcagacgctg gtccacttca 1200
   tcaacccgga aacggtgccc aagccctgct gtgcgcccac gcagctcaat gccatctccg 1260
   tcctctactt cgatgacagc tccaacgtca tcctgaagaa atacagaaac atggtggtcc 1320
   gggcctgtgg ctgccac                                                1337
<210> 6
   <211> 424
   <212> PRT
   <213> Artificial Sequence
<220>
   <223> cDNA encoding a hBMP-7 precursor derived variant peptide chain comprising the delta S293-R299 and R314S mutations.
<400> 6
   Met His Val Arg Ser Leu Arg Ala Ala Ala Pro His Ser Phe Val Ala
   1 5 10 15
   Leu Trp Ala Pro Leu Phe Leu Leu Arg Ser Ala Leu Ala Asp Phe Ser
   20 25 30
   Leu Asp Asn Glu Val His Ser Ser Phe Ile His Arg Arg Leu Arg Ser
   35 40 45
   Gln Glu Arg Arg Glu Met Gln Arg Glu Ile Leu Ser Ile Leu Gly Leu
   50 55 60
   Pro His Arg Pro Arg Pro His Leu Gln Gly Lys His Asn Ser Ala Pro
   65 70 75 80
   Met Phe Met Leu Asp Leu Tyr Asn Ala Met Ala Val Glu Glu Gly Gly
   85 90 95
   Gly Pro Gly Gly Gln Gly Phe Ser Tyr Pro Tyr Lys Ala Val Phe Ser
   100 105 110
   Thr Gln Gly Pro Pro Leu Ala Ser Leu Gln Asp Ser His Phe Leu Thr
   115 120 125
   Asp Ala Asp Met Val Met Ser Phe Val Asn Leu Val Glu His Asp Lys
   130 135 140
   Glu Phe Phe His Pro Arg Tyr His His Arg Glu Phe Arg Phe Asp Leu
   145 150 155 160
   Ser Lys Ile Pro Glu Gly Glu Ala Val Thr Ala Ala Glu Phe Arg Ile
   165 170 175
   Tyr Lys Asp Tyr Ile Arg Glu Arg Phe Asp Asn Glu Thr Phe Arg Ile
   180 185 190
   Ser Val Tyr Gln Val Leu Gln Glu His Leu Gly Arg Glu Ser Asp Leu
   195 200 205
   Phe Leu Leu Asp Ser Arg Thr Leu Trp Ala Ser Glu Glu Gly Trp Leu
   210 215 220
   Val Phe Asp Ile Thr Ala Thr Ser Asn His Trp Val Val Asn Pro Arg
   225 230 235 240
   His Asn Leu Gly Leu Gln Leu Ser Val Glu Thr Leu Asp Gly Gln Ser
   245 250 255
   Ile Asn Pro Lys Leu Ala Gly Leu Ile Gly Arg His Gly Pro Gln Asn
   260 265 270
   Lys Gln Pro Phe Met Val Ala Phe Phe Lys Ala Thr Glu Val His Phe
   275 280 285
   Arg Ser Ile Arg Ser Gln Asn Arg Ser Lys Thr Pro Lys Asn Gln Glu
   290 295 300
   Ala Leu Ser Met Ala Asn Val Ala Glu Asn Ser Ser Ser Asp Gln Arg
   305 310 315 320
   Gln Ala Cys Lys Lys His Glu Leu Tyr Val Ser Phe Arg Asp Leu Gly
   325 330 335
   Trp Gln Asp Trp Ile Ile Ala Pro Glu Gly Tyr Ala Ala Tyr Tyr Cys
   340 345 350
   Glu Gly Glu Cys Ala Phe Pro Leu Asn Ser Tyr Met Asn Ala Thr Asn
   355 360 365
   His Ala Ile Val Gln Thr Leu Val His Phe Ile Asn Pro Glu Thr Val
   370 375 380
   Pro Lys Pro Cys Cys Ala Pro Thr Gln Leu Asn Ala Ile Ser Val Leu
   385 390 395 400
   Tyr Phe Asp Asp Ser Ser Asn Val Ile Leu Lys Lys Tyr Arg Asn Met
   405 410 415
   Val Val Arg Ala Cys Gly Cys His
   420
<210> 7
   <211> 1227
   <212> DNA
   <213> Artificial Sequence
<220>
   <223> cDNA encoding a hBMP-7 precursor derived variant peptide chain comprising the delta S293 and M314 mutations.
<400> 7
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggatgg ccaacgtggc agagaacagc 900
   agcagcgacc agaggcaggc ctgtaagaag cacgagctgt atgtcagctt ccgagacctg 960
   ggctggcagg actggatcat cgcgcctgaa ggctacgccg cctactactg tgagggggag 1020
   tgtgccttcc ctctgaactc ctacatgaac gccaccaacc acgccatcgt gcagacgctg 1080
   gtccacttca tcaacccgga aacggtgccc aagccctgct gtgcgcccac gcagctcaat 1140
   gccatctccg tcctctactt cgatgacagc tccaacgtca tcctgaagaa atacagaaac 1200
   atggtggtcc gggcctgtgg ctgccac                                     1227
<210> 8
   <211> 409
   <212> PRT
   <213> Artificial Sequence
<220>
   <223> cDNA encoding a hBMP-7 precursor derived variant peptide chain comprising the delta S293 and M314 mutations.
<400> 8
   Met His Val Arg Ser Leu Arg Ala Ala Ala Pro His Ser Phe Val Ala
   1               5                   10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Met Ala Asn Val Ala Glu Asn Ser Ser Ser Asp Gln
       290                 295                 300
   Arg Gln Ala Cys Lys Lys His Glu Leu Tyr Val Ser Phe Arg Asp Leu
   305                 310                 315                 320
   Gly Trp Gln Asp Trp Ile Ile Ala Pro Glu Gly Tyr Ala Ala Tyr Tyr
                   325                 330                 335
   Cys Glu Gly Glu Cys Ala Phe Pro Leu Asn Ser Tyr Met Asn Ala Thr
               340                 345                 350
   Asn His Ala Ile Val Gln Thr Leu Val His Phe Ile Asn Pro Glu Thr
           355                 360                 365
   Val Pro Lys Pro Cys Cys Ala Pro Thr Gln Leu Asn Ala Ile Ser Val
       370                 375                 380
   Leu Tyr Phe Asp Asp Ser Ser Asn Val Ile Leu Lys Lys Tyr Arg Asn
   385                 390                 395                 400
   Met Val Val Arg Ala Cys Gly Cys His
                   405
<210> 9
   <211> 1224
   <212> DNA
   <213> Artificial Sequence
<220>
   <223> cDNA encoding a hBMP-7 precursor derived variant peptide chain comprising the delta S293 and M315 mutations.
<400> 9
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccgggcca acgtggcaga gaacagcagc 900
   agcgaccaga ggcaggcctg taagaagcac gagctgtatg tcagcttccg agacctgggc 960
   tggcaggact ggatcatcgc gcctgaaggc tacgccgcct actactgtga gggggagtgt 1020
   gccttccctc tgaactccta catgaacgcc accaaccacg ccatcgtgca gacgctggtc 1080
   cacttcatca acccggaaac ggtgcccaag ccctgctgtg cgcccacgca gctcaatgcc 1140
   atctccgtcc tctacttcga tgacagctcc aacgtcatcc tgaagaaata cagaaacatg 1200
   gtggtccggg cctgtggctg ccac                                        1224
<210> 10
   <211> 408
   <212> PRT
   <213> Artificial Sequence
<220>
   <223> cDNA encoding a hBMP-7 precursor derived variant peptide chain comprising the delta S293 and M315 mutations.
<400> 10
   Met His Val Arg Ser Leu Arg Ala Ala Ala Pro His Ser Phe Val Ala
   1               5                   10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ala Asn Val Ala Glu Asn Ser Ser Ser Asp Gln Arg
       290                 295                 300
   Gln Ala Cys Lys Lys His Glu Leu Tyr Val Ser Phe Arg Asp Leu Gly
   305                 310                 315                 320
   Trp Gln Asp Trp Ile Ile Ala Pro Glu Gly Tyr Ala Ala Tyr Tyr Cys
                   325                 330                 335
   Glu Gly Glu Cys Ala Phe Pro Leu Asn Ser Tyr Met Asn Ala Thr Asn
               340                 345                 350
   His Ala Ile Val Gln Thr Leu Val His Phe Ile Asn Pro Glu Thr Val
           355                 360                 365
   Pro Lys Pro Cys Cys Ala Pro Thr Gln Leu Asn Ala Ile Ser Val Leu
       370                 375                 380
   Tyr Phe Asp Asp Ser Ser Asn Val Ile Leu Lys Lys Tyr Arg Asn Met
   385                 390                 395                 400
   Val Val Arg Ala Cys Gly Cys His
                   405
<210> 11
   <211> 1293
   <212> DNA
   <213> Artificial Sequence
<220>
   <223> cDNA encoding a hBMP-7 precursor derived variant peptide chain comprising the delta R299S, R314S, and L407K mutations.
<400> 11
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggtcca cggggagcaa acagagcagc 900
   cagaaccgct ccaagacgcc caagaaccag gaagccctga gcatggccaa cgtggcagag 960
   aacagcagca gcgaccagag gcaggcctgt aagaagcacg agctgtatgt cagcttccga 1020
   gacctgggct ggcaggactg gatcatcgcg cctgaaggct acgccgccta ctactgtgag 1080
   ggggagtgtg ccttccctct gaactcctac atgaacgcca ccaaccacgc catcgtgcag 1140
   acgctggtcc acttcatcaa cccggaaacg gtgcccaagc cctgctgtgc gcccacgcag 1200
   ctcaatgcca tctccgtcaa gtacttcgat gacagctcca acgtcatcct gaagaaatac 1260
   agaaacatgg tggtccgggc ctgtggctgc cac                              1293
<210> 12
   <211> 431
   <212> PRT
   <213> Artificial Sequence
<220>
   <223> cDNA encoding a hBMP-7 precursor derived variant peptide chain comprising the delta R299S, R314S, and L407K mutations.
<400> 12
   Met His Val Arg Ser Leu Arg Ala Ala Ala Pro His Ser Phe Val Ala
   1               5                   10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Ser Ser Gln Asn Arg Ser
       290                 295                 300
   Lys Thr Pro Lys Asn Gln Glu Ala Leu Ser Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Val His
   370 375 380
   Phe Ile Asn Pro Glu Thr Val Pro Lys Pro Cys Cys Ala Pro Thr Gln
   385 390 395 400
   Leu Asn Ala Ile Ser Val Lys Tyr Phe Asp Asp Ser Ser Asn Val Ile
   405 410 415
   Leu Lys Lys Tyr Arg Asn Met Val Val Arg Ala Cys Gly Cys His
   420 425 430
<210> 13
   <211> 1293
   <212> DNA
   <213> Artificial Sequence
<220>
   <223> cDNA encoding a hBMP-7 precursor derived variant peptide chain comprising the delta R299S, R314S, and F409K mutations.
<400> 13
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggtcca cggggagcaa acagagcagc 900
   cagaaccgct ccaagacgcc caagaaccag gaagccctga gcatggccaa cgtggcagag 960
   aacagcagca gcgaccagag gcaggcctgt aagaagcacg agctgtatgt cagcttccga 1020
   gacctgggct ggcaggactg gatcatcgcg cctgaaggct acgccgccta ctactgtgag 1080
   ggggagtgtg ccttccctct gaactcctac atgaacgcca ccaaccacgc catcgtgcag 1140
   acgctggtcc acttcatcaa cccggaaacg gtgcccaagc cctgctgtgc gcccacgcag 1200
   ctcaatgcca tctccgtcct ctacaaggat gacagctcca acgtcatcct gaagaaatac 1260
   agaaacatgg tggtccgggc ctgtggctgc cac 1293
<210> 14
   <211> 431
   <212> PRT
   <213> Artificial Sequence
<220>
   <223> cDNA encoding a hBMP-7 precursor derived variant peptide chain comprising the delta R299S, R314S, and F409K mutations.
<400> 14
   Met His Val Arg Ser Leu Arg Ala Ala Ala Pro His Ser Phe Val Ala
   1               5                   10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Ser Ser Gln Asn Arg Ser
       290                 295                 300
   Lys Thr Pro Lys Asn Gln Glu Ala Leu Ser Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Val His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Leu Tyr Lys Asp Asp Ser Ser Asn Val Ile
                   405                 410                 415
   Leu Lys Lys Tyr Arg Asn Met Val Val Arg Ala Cys Gly Cys His
               420                 425                 430
<210> 15
   <211> 1293
   <212> DNA
   <213> Artificial Sequence
<220>
   <223> cDNA encoding a hBMP-7 precursor derived variant peptide chain comprising the delta R299S, R314S, and F417R mutations.
<400> 15
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggtcca cggggagcaa acagagcagc 900
   cagaaccgct ccaagacgcc caagaaccag gaagccctga gcatggccaa cgtggcagag 960
   aacagcagca gcgaccagag gcaggcctgt aagaagcacg agctgtatgt cagcttccga 1020
   gacctgggct ggcaggactg gatcatcgcg cctgaaggct acgccgccta ctactgtgag 1080
   ggggagtgtg ccttccctct gaactcctac atgaacgcca ccaaccacgc catcgtgcag 1140
   acgctggtcc acttcatcaa cccggaaacg gtgcccaagc cctgctgtgc gcccacgcag 1200
   ctcaatgcca tctccgtcct ctacttcgat gacagctcca acgtcatccg gaagaaatac 1260
   agaaacatgg tggtccgggc ctgtggctgc cac 1293
<210> 16
   <211> 431
   <212> PRT
   <213> Artificial Sequence
<220>
   <223> cDNA encoding a hBMP-7 precursor derived variant peptide chain comprising the delta R299S, R314S, and F417R mutations.
<400> 16
   Met His Val Arg Ser Leu Arg Ala Ala Ala Pro His Ser Phe Val Ala
   1 5 10 15
   Leu Trp Ala Pro Leu Phe Leu Leu Arg Ser Ala Leu Ala Asp Phe Ser
   20 25 30
   Leu Asp Asn Glu Val His Ser Ser Phe Ile His Arg Arg Leu Arg Ser
   35 40 45
   Gln Glu Arg Arg Glu Met Gln Arg Glu Ile Leu Ser Ile Leu Gly Leu
   50 55 60
   Pro His Arg Pro Arg Pro His Leu Gln Gly Lys His Asn Ser Ala Pro
   65 70 75 80
   Met Phe Met Leu Asp Leu Tyr Asn Ala Met Ala Val Glu Glu Gly Gly
   85 90 95
   Gly Pro Gly Gly Gln Gly Phe Ser Tyr Pro Tyr Lys Ala Val Phe Ser
   100 105 110
   Thr Gln Gly Pro Pro Leu Ala Ser Leu Gln Asp Ser His Phe Leu Thr
   115 120 125
   Asp Ala Asp Met Val Met Ser Phe Val Asn Leu Val Glu His Asp Lys
   130 135 140
   Glu Phe Phe His Pro Arg Tyr His His Arg Glu Phe Arg Phe Asp Leu
   145 150 155 160
   Ser Lys Ile Pro Glu Gly Glu Ala Val Thr Ala Ala Glu Phe Arg Ile
   165 170 175
   Tyr Lys Asp Tyr Ile Arg Glu Arg Phe Asp Asn Glu Thr Phe Arg Ile
   180 185 190
   Ser Val Tyr Gln Val Leu Gln Glu His Leu Gly Arg Glu Ser Asp Leu
   195 200 205
   Phe Leu Leu Asp Ser Arg Thr Leu Trp Ala Ser Glu Glu Gly Trp Leu
   210 215 220
   Val Phe Asp Ile Thr Ala Thr Ser Asn His Trp Val Val Asn Pro Arg
   225 230 235 240
   His Asn Leu Gly Leu Gln Leu Ser Val Glu Thr Leu Asp Gly Gln Ser
   245 250 255
   Ile Asn Pro Lys Leu Ala Gly Leu Ile Gly Arg His Gly Pro Gln Asn
   260 265 270
   Lys Gln Pro Phe Met Val Ala Phe Phe Lys Ala Thr Glu Val His Phe
   275 280 285
   Arg Ser Ile Arg Ser Thr Gly Ser Lys Gln Ser Ser Gln Asn Arg Ser
   290 295 300
   Lys Thr Pro Lys Asn Gln Glu Ala Leu Ser Met Ala Asn Val Ala Glu
   305 310 315 320
   Asn Ser Ser Ser Asp Gln Arg Gln Ala Cys Lys Lys His Glu Leu Tyr
   325 330 335
   Val Ser Phe Arg Asp Leu Gly Trp Gln Asp Trp Ile Ile Ala Pro Glu
   340 345 350
   Gly Tyr Ala Ala Tyr Tyr Cys Glu Gly Glu Cys Ala Phe Pro Leu Asn
   355 360 365
   Ser Tyr Met Asn Ala Thr Asn His Ala Ile Val Gln Thr Leu Val His
   370 375 380
   Phe Ile Asn Pro Glu Thr Val Pro Lys Pro Cys Cys Ala Pro Thr Gln
   385 390 395 400
   Leu Asn Ala Ile Ser Val Leu Tyr Phe Asp Asp Ser Ser Asn Val Ile
   405 410 415
   Arg Lys Lys Tyr Arg Asn Met Val Val Arg Ala Cys Gly Cys His
   420 425 430
<210> 17
   <211> 1293
   <212> DNA
   <213> Artificial Sequence
<220>
   <223> cDNA encoding a hBMP-7 precursor derived variant peptide chain comprising the delta R299S, R314S, and L382K mutations.
<400> 17
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggtcca cggggagcaa acagagcagc 900
   cagaaccgct ccaagacgcc caagaaccag gaagccctga gcatggccaa cgtggcagag 960
   aacagcagca gcgaccagag gcaggcctgt aagaagcacg agctgtatgt cagcttccga 1020
   gacctgggct ggcaggactg gatcatcgcg cctgaaggct acgccgccta ctactgtgag 1080
   ggggagtgtg ccttccctct gaactcctac atgaacgcca ccaaccacgc catcgtgcag 1140
   acgaaggtcc acttcatcaa cccggaaacg gtgcccaagc cctgctgtgc gcccacgcag 1200
   ctcaatgcca tctccgtcct ctacttcgat gacagctcca acgtcatcct gaagaaatac 1260
   agaaacatgg tggtccgggc ctgtggctgc cac                              1293
<210> 18
   <211> 431
   <212> PRT
   <213> Artificial Sequence
<220>
   <223> cDNA encoding a hBMP-7 precursor derived variant peptide chain comprising the delta R299S, R314S, and L382K mutations.
<400> 18
   Met His Val Arg Ser Leu Arg Ala Ala Ala Pro His Ser Phe Val Ala
   1 5 10 15
   Leu Trp Ala Pro Leu Phe Leu Leu Arg Ser Ala Leu Ala Asp Phe Ser
   20 25 30
   Leu Asp Asn Glu Val His Ser Ser Phe Ile His Arg Arg Leu Arg Ser
   35 40 45
   Gln Glu Arg Arg Glu Met Gln Arg Glu Ile Leu Ser Ile Leu Gly Leu
   50 55 60
   Pro His Arg Pro Arg Pro His Leu Gln Gly Lys His Asn Ser Ala Pro
   65 70 75 80
   Met Phe Met Leu Asp Leu Tyr Asn Ala Met Ala Val Glu Glu Gly Gly
   85 90 95
   Gly Pro Gly Gly Gln Gly Phe Ser Tyr Pro Tyr Lys Ala Val Phe Ser
   100 105 110
   Thr Gln Gly Pro Pro Leu Ala Ser Leu Gln Asp Ser His Phe Leu Thr
   115 120 125
   Asp Ala Asp Met Val Met Ser Phe Val Asn Leu Val Glu His Asp Lys
   130 135 140
   Glu Phe Phe His Pro Arg Tyr His His Arg Glu Phe Arg Phe Asp Leu
   145 150 155 160
   Ser Lys Ile Pro Glu Gly Glu Ala Val Thr Ala Ala Glu Phe Arg Ile
   165 170 175
   Tyr Lys Asp Tyr Ile Arg Glu Arg Phe Asp Asn Glu Thr Phe Arg Ile
   180 185 190
   Ser Val Tyr Gln Val Leu Gln Glu His Leu Gly Arg Glu Ser Asp Leu
   195 200 205
   Phe Leu Leu Asp Ser Arg Thr Leu Trp Ala Ser Glu Glu Gly Trp Leu
   210 215 220
   Val Phe Asp Ile Thr Ala Thr Ser Asn His Trp Val Val Asn Pro Arg
   225 230 235 240
   His Asn Leu Gly Leu Gln Leu Ser Val Glu Thr Leu Asp Gly Gln Ser
   245 250 255
   Ile Asn Pro Lys Leu Ala Gly Leu Ile Gly Arg His Gly Pro Gln Asn
   260 265 270
   Lys Gln Pro Phe Met Val Ala Phe Phe Lys Ala Thr Glu Val His Phe
   275 280 285
   Arg Ser Ile Arg Ser Thr Gly Ser Lys Gln Ser Ser Gln Asn Arg Ser
   290 295 300
   Lys Thr Pro Lys Asn Gln Glu Ala Leu Ser Met Ala Asn Val Ala Glu
   305 310 315 320
   Asn Ser Ser Ser Asp Gln Arg Gln Ala Cys Lys Lys His Glu Leu Tyr
   325 330 335
   Val Ser Phe Arg Asp Leu Gly Trp Gln Asp Trp Ile Ile Ala Pro Glu
   340 345 350
   Gly Tyr Ala Ala Tyr Tyr Cys Glu Gly Glu Cys Ala Phe Pro Leu Asn
   355 360 365
   Ser Tyr Met Asn Ala Thr Asn His Ala Ile Val Gln Thr Lys Val His
   370 375 380
   Phe Ile Asn Pro Glu Thr Val Pro Lys Pro Cys Cys Ala Pro Thr Gln
   385 390 395 400
   Leu Asn Ala Ile Ser Val Leu Tyr Phe Asp Asp Ser Ser Asn Val Ile
   405 410 415
   Leu Lys Lys Tyr Arg Asn Met Val Val Arg Ala Cys Gly Cys His
   420 425 430
<210> 19
   <211> 1293
   <212> DNA
   <213> Artificial Sequence
<220>
   <223> cDNA encoding a hBMP-7 precursor derived variant peptide chain comprising the delta R299S, R314S, and L382N mutations.
<400> 19
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggtcca cggggagcaa acagagcagc 900
   cagaaccgct ccaagacgcc caagaaccag gaagccctga gcatggccaa cgtggcagag 960
   aacagcagca gcgaccagag gcaggcctgt aagaagcacg agctgtatgt cagcttccga 1020
   gacctgggct ggcaggactg gatcatcgcg cctgaaggct acgccgccta ctactgtgag 1080
   ggggagtgtg ccttccctct gaactcctac atgaacgcca ccaaccacgc catcgtgcag 1140
   acgaacgtcc acttcatcaa cccggaaacg gtgcccaagc cctgctgtgc gcccacgcag 1200
   ctcaatgcca tctccgtcct ctacttcgat gacagctcca acgtcatcct gaagaaatac 1260
   agaaacatgg tggtccgggc ctgtggctgc cac                               1293
<210> 20
   <211> 431
   <212> PRT
   <213> Artificial Sequence
<220>
   <223> cDNA encoding a hBMP-7 precursor derived variant peptide chain comprising the delta R299S, R314S, and L382N mutations.
<400> 20
   Met His Val Arg Ser Leu Arg Ala Ala Ala Pro His Ser Phe Val Ala
   1               5                   10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Ser Ser Gln Asn Arg Ser
       290                 295                 300
   Lys Thr Pro Lys Asn Gln Glu Ala Leu Ser Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Asn Val His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Leu Tyr Phe Asp Asp Ser Ser Asn Val Ile
                   405                 410                 415
   Leu Lys Lys Tyr Arg Asn Met Val Val Arg Ala Cys Gly Cys His
               420                 425                 430
<210> 21
   <211> 1293
   <212> DNA
   <213> Artificial Sequence
<220>
   <223> cDNA encoding a hBMP-7 precursor derived variant peptide chain comprising the delta R299S, R314S, and V383K mutations.
<400> 21
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggtcca cggggagcaa acagagcagc 900
   cagaaccgct ccaagacgcc caagaaccag gaagccctga gcatggccaa cgtggcagag 960
   aacagcagca gcgaccagag gcaggcctgt aagaagcacg agctgtatgt cagcttccga 1020
   gacctgggct ggcaggactg gatcatcgcg cctgaaggct acgccgccta ctactgtgag 1080
   ggggagtgtg ccttccctct gaactcctac atgaacgcca ccaaccacgc catcgtgcag 1140
   acgctgaagc acttcatcaa cccggaaacg gtgcccaagc cctgctgtgc gcccacgcag 1200
   ctcaatgcca tctccgtcct ctacttcgat gacagctcca acgtcatcct gaagaaatac 1260
   agaaacatgg tggtccgggc ctgtggctgc cac                              1293
<210> 22
   <211> 431
   <212> PRT
   <213> Artificial Sequence
<220>
   <223> cDNA encoding a hBMP-7 precursor derived variant peptide chain comprising the delta R299S, R314S, and V383K mutations.
<400> 22
   Met His Val Arg Ser Leu Arg Ala Ala Ala Pro His Ser Phe Val Ala
    1               5                  10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Ser Ser Gln Asn Arg Ser
       290                 295                 300
   Lys Thr Pro Lys Asn Gln Glu Ala Leu Ser Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Lys His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Leu Tyr Phe Asp Asp Ser Ser Asn Val Ile
                   405                 410                 415
   Leu Lys Lys Tyr Arg Asn Met Val Val Arg Ala Cys Gly Cys His
               420                 425                 430
<210> 23
   <211> 1293
   <212> DNA
   <213> Artificial Sequence
<220>
   <223> cDNA encoding a hBMP-7 precursor derived variant peptide chain comprising the delta R299S, R314S, and I386R mutations.
<400> 23
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggtcca cggggagcaa acagagcagc 900
   cagaaccgct ccaagacgcc caagaaccag gaagccctga gcatggccaa cgtggcagag 960
   aacagcagca gcgaccagag gcaggcctgt aagaagcacg agctgtatgt cagcttccga 1020
   gacctgggct ggcaggactg gatcatcgcg cctgaaggct acgccgccta ctactgtgag 1080
   ggggagtgtg ccttccctct gaactcctac atgaacgcca ccaaccacgc catcgtgcag 1140
   acgctggtcc acttcaggaa cccggaaacg gtgcccaagc cctgctgtgc gcccacgcag 1200
   ctcaatgcca tctccgtcct ctacttcgat gacagctcca acgtcatcct gaagaaatac 1260
   agaaacatgg tggtccgggc ctgtggctgc cac 1293
<210> 24
   <211> 431
   <212> PRT
   <213> Artificial Sequence
<220>
   <223> cDNA encoding a hBMP-7 precursor derived variant peptide chain comprising the delta R299S, R314S, and I386R mutations.
<400> 24
   Met His Val Arg Ser Leu Arg Ala Ala Ala Pro His Ser Phe Val Ala
   1 5 10 15
   Leu Trp Ala Pro Leu Phe Leu Leu Arg Ser Ala Leu Ala Asp Phe Ser
   20 25 30
   Leu Asp Asn Glu Val His Ser Ser Phe Ile His Arg Arg Leu Arg Ser
   35 40 45
   Gln Glu Arg Arg Glu Met Gln Arg Glu Ile Leu Ser Ile Leu Gly Leu
   50 55 60
   Pro His Arg Pro Arg Pro His Leu Gln Gly Lys His Asn Ser Ala Pro
   65 70 75 80
   Met Phe Met Leu Asp Leu Tyr Asn Ala Met Ala Val Glu Glu Gly Gly
   85 90 95
   Gly Pro Gly Gly Gln Gly Phe Ser Tyr Pro Tyr Lys Ala Val Phe Ser
   100 105 110
   Thr Gln Gly Pro Pro Leu Ala Ser Leu Gln Asp Ser His Phe Leu Thr
   115 120 125
   Asp Ala Asp Met Val Met Ser Phe Val Asn Leu Val Glu His Asp Lys
   130 135 140
   Glu Phe Phe His Pro Arg Tyr His His Arg Glu Phe Arg Phe Asp Leu
   145 150 155 160
   Ser Lys Ile Pro Glu Gly Glu Ala Val Thr Ala Ala Glu Phe Arg Ile
   165 170 175
   Tyr Lys Asp Tyr Ile Arg Glu Arg Phe Asp Asn Glu Thr Phe Arg Ile
   180 185 190
   Ser Val Tyr Gln Val Leu Gln Glu His Leu Gly Arg Glu Ser Asp Leu
   195 200 205
   Phe Leu Leu Asp Ser Arg Thr Leu Trp Ala Ser Glu Glu Gly Trp Leu
   210 215 220
   Val Phe Asp Ile Thr Ala Thr Ser Asn His Trp Val Val Asn Pro Arg
   225 230 235 240
   His Asn Leu Gly Leu Gln Leu Ser Val Glu Thr Leu Asp Gly Gln Ser
   245 250 255
   Ile Asn Pro Lys Leu Ala Gly Leu Ile Gly Arg His Gly Pro Gln Asn
   260 265 270
   Lys Gln Pro Phe Met Val Ala Phe Phe Lys Ala Thr Glu Val His Phe
   275 280 285
   Arg Ser Ile Arg Ser Thr Gly Ser Lys Gln Ser Ser Gln Asn Arg Ser
   290 295 300
   Lys Thr Pro Lys Asn Gln Glu Ala Leu Ser Met Ala Asn Val Ala Glu
   305 310 315 320
   Asn Ser Ser Ser Asp Gln Arg Gln Ala Cys Lys Lys His Glu Leu Tyr
   325 330 335
   Val Ser Phe Arg Asp Leu Gly Trp Gln Asp Trp Ile Ile Ala Pro Glu
   340 345 350
   Gly Tyr Ala Ala Tyr Tyr Cys Glu Gly Glu Cys Ala Phe Pro Leu Asn
   355 360 365
   Ser Tyr Met Asn Ala Thr Asn His Ala Ile Val Gln Thr Leu Val His
   370 375 380
   Phe Arg Asn Pro Glu Thr Val Pro Lys Pro Cys Cys Ala Pro Thr Gln
   385 390 395 400
   Leu Asn Ala Ile Ser Val Leu Tyr Phe Asp Asp Ser Ser Asn Val Ile
   405 410 415
   Leu Lys Lys Tyr Arg Asn Met Val Val Arg Ala Cys Gly Cys His
   420 425 430
<210> 25
   <211> 1293
   <212> DNA
   <213> Artificial Sequence
<220>
   <223> cDNA encoding a hBMP-7 precursor derived variant peptide chain comprising the delta R299S, R314S, and I386R mutations.
<400> 25
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggtcca cggggagcaa acagagcagc 900
   cagaaccgct ccaagacgcc caagaaccag gaagccctga gcatggccaa cgtggcagag 960
   aacagcagca gcgaccagag gcaggcctgt aagaagcacg agctgtatgt cagcttccga 1020
   gacctgggct ggcaggactg gatcatcgcg cctgaaggct acgccgccta ctactgtgag 1080
   ggggagtgtg ccttccctct gaactcctac atgaacgcca ccaaccacgc catcgtgcag 1140
   acgctggtcc acttcaacaa cccggaaacg gtgcccaagc cctgctgtgc gcccacgcag 1200
   ctcaatgcca tctccgtcct ctacttcgat gacagctcca acgtcatcct gaagaaatac 1260
   agaaacatgg tggtccgggc ctgtggctgc cac 1293
<210> 26
   <211> 431
   <212> PRT
   <213> Artificial Sequence
<220>
   <223> cDNA encoding a hBMP-7 precursor derived variant peptide chain comprising the delta R299S, R314S, and I386R mutations.
<400> 26
   Met His Val Arg Ser Leu Arg Ala Ala Ala Pro His Ser Phe Val Ala
   1 5 10 15
   Leu Trp Ala Pro Leu Phe Leu Leu Arg Ser Ala Leu Ala Asp Phe Ser
   20 25 30
   Leu Asp Asn Glu Val His Ser Ser Phe Ile His Arg Arg Leu Arg Ser
   35 40 45
   Gln Glu Arg Arg Glu Met Gln Arg Glu Ile Leu Ser Ile Leu Gly Leu
   50 55 60
   Pro His Arg Pro Arg Pro His Leu Gln Gly Lys His Asn Ser Ala Pro
   65 70 75 80
   Met Phe Met Leu Asp Leu Tyr Asn Ala Met Ala Val Glu Glu Gly Gly
   85 90 95
   Gly Pro Gly Gly Gln Gly Phe Ser Tyr Pro Tyr Lys Ala Val Phe Ser
   100 105 110
   Thr Gln Gly Pro Pro Leu Ala Ser Leu Gln Asp Ser His Phe Leu Thr
   115 120 125
   Asp Ala Asp Met Val Met Ser Phe Val Asn Leu Val Glu His Asp Lys
   130 135 140
   Glu Phe Phe His Pro Arg Tyr His His Arg Glu Phe Arg Phe Asp Leu
   145 150 155 160
   Ser Lys Ile Pro Glu Gly Glu Ala Val Thr Ala Ala Glu Phe Arg Ile
   165 170 175
   Tyr Lys Asp Tyr Ile Arg Glu Arg Phe Asp Asn Glu Thr Phe Arg Ile
   180 185 190
   Ser Val Tyr Gln Val Leu Gln Glu His Leu Gly Arg Glu Ser Asp Leu
   195 200 205
   Phe Leu Leu Asp Ser Arg Thr Leu Trp Ala Ser Glu Glu Gly Trp Leu
   210 215 220
   Val Phe Asp Ile Thr Ala Thr Ser Asn His Trp Val Val Asn Pro Arg
   225 230 235 240
   His Asn Leu Gly Leu Gln Leu Ser Val Glu Thr Leu Asp Gly Gln Ser
   245 250 255
   Ile Asn Pro Lys Leu Ala Gly Leu Ile Gly Arg His Gly Pro Gln Asn
   260 265 270
   Lys Gln Pro Phe Met Val Ala Phe Phe Lys Ala Thr Glu Val His Phe
   275 280 285
   Arg Ser Ile Arg Ser Thr Gly Ser Lys Gln Ser Ser Gln Asn Arg Ser
   290 295 300
   Lys Thr Pro Lys Asn Gln Glu Ala Leu Ser Met Ala Asn Val Ala Glu
   305 310 315 320
   Asn Ser Ser Ser Asp Gln Arg Gln Ala Cys Lys Lys His Glu Leu Tyr
   325 330 335
   Val Ser Phe Arg Asp Leu Gly Trp Gln Asp Trp Ile Ile Ala Pro Glu
   340 345 350
   Gly Tyr Ala Ala Tyr Tyr Cys Glu Gly Glu Cys Ala Phe Pro Leu Asn
   355 360 365
   Ser Tyr Met Asn Ala Thr Asn His Ala Ile Val Gln Thr Leu Val His
   370 375 380
   Phe Asn Asn Pro Glu Thr Val Pro Lys Pro Cys Cys Ala Pro Thr Gln
   385 390 395 400
   Leu Asn Ala Ile Ser Val Leu Tyr Phe Asp Asp Ser Ser Asn Val Ile
   405 410 415
   Leu Lys Lys Tyr Arg Asn Met Val Val Arg Ala Cys Gly Cys His
   420 425 430
<210> 27
   <211> 1293
   <212> DNA
   <213> Artificial Sequence
<220>
   <223> cDNA encoding a hBMP-7 precursor derived variant peptide chain comprising the delta R299S, R314S, and V383K mutations.
<400> 27
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggtcca cggggagcaa acagagcagc 900
   cagaaccgct ccaagacgcc caagaaccag gaagccctga gcatggccaa cgtggcagag 960
   aacagcagca gcgaccagag gcaggcctgt aagaagcacg agctgtatgt cagcttccga 1020
   gacctgggct ggcaggactg gatcatcgcg cctgaaggct acgccgccta ctactgtgag 1080
   ggggagtgtg ccttccctct gaactcctac atgaacgcca ccaaccacgc catcgtgcag 1140
   acgctgaagc acttcatcaa cccggaaacg gtgcccaagc cctgctgtgc gcccacgcag 1200
   ctcaatgcca tctccgtcaa gtacaaggat gacagctcca acgtcatcct gaagaaatac 1260
   agaaacatgg tggtccgggc ctgtggctgc cac                              1293
<210> 28
   <211> 431
   <212> PRT
   <213> Artificial Sequence
<220>
   <223> cDNA encoding a hBMP-7 precursor derived variant peptide chain comprising the delta R299S, R314S, and V383K , L407K and F409K mutations.
<400> 28
   Met His Val Arg Ser Leu Arg Ala Ala Ala Pro His Ser Phe Val Ala
   1               5                   10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Ser Ser Gln Asn Arg Ser
       290                 295                 300
   Lys Thr Pro Lys Asn Gln Glu Ala Leu Ser Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Lys His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Lys Tyr Lys Asp Asp Ser Ser Asn Val Ile
                   405                 410                 415
   Leu Lys Lys Tyr Arg Asn Met Val Val Arg Ala Cys Gly Cys His
               420                 425                 430
<210> 29
   <211> 1293
   <212> DNA
   <213> Artificial Sequence
<220>
   <223> cDNA encoding a hBMP-7 precursor derived variant peptide chain comprising the delta R299S, R314S, and W347D mutations.
<400> 29
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggtcca cggggagcaa acagagcagc 900
   cagaaccgct ccaagacgcc caagaaccag gaagccctga gcatggccaa cgtggcagag 960
   aacagcagca gcgaccagag gcaggcctgt aagaagcacg agctgtatgt cagcttccga 1020
   gacctgggct ggcaggacga catcatcgcg cctgaaggct acgccgccta ctactgtgag 1080
   ggggagtgtg ccttccctct gaactcctac atgaacgcca ccaaccacgc catcgtgcag 1140
   acgctggtcc acttcatcaa cccggaaacg gtgcccaagc cctgctgtgc gcccacgcag 1200
   ctcaatgcca tctccgtcct ctacttcgat gacagctcca acgtcatcct gaagaaatac 1260
   agaaacatgg tggtccgggc ctgtggctgc cac                               1293
<210> 30
   <211> 431
   <212> PRT
   <213> Artificial Sequence
<220>
   <223> cDNA encoding a hBMP-7 precursor derived variant peptide chain comprising the delta R299S, R314S, and W347D mutations.
<400> 30
   Met His Val Arg Ser Leu Arg Ala Ala Ala Pro His Ser Phe Val Ala
   1               5                   10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Ser Ser Gln Asn Arg Ser
       290                 295                 300
   Lys Thr Pro Lys Asn Gln Glu Ala Leu Ser Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Asp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Val His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Leu Tyr Phe Asp Asp Ser Ser Asn Val Ile
                   405                 410                 415
   Leu Lys Lys Tyr Arg Asn Met Val Val Arg Ala Cys Gly Cys His
               420                 425                 430
<210> 31
   <211> 1293
   <212> DNA
   <213> Artificial Sequence
<220>
   <223> cDNA encoding a hBMP-7 precursor derived variant peptide chain comprising the delta R299S, R314S, and R421E, N422D, and R426E mutations.
<400> 31
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggtcca cggggagcaa acagagcagc 900
   cagaaccgct ccaagacgcc caagaaccag gaagccctga gcatggccaa cgtggcagag 960
   aacagcagca gcgaccagag gcaggcctgt aagaagcacg agctgtatgt cagcttccga 1020
   gacctgggct ggcaggactg gatcatcgcg cctgaaggct acgccgccta ctactgtgag 1080
   ggggagtgtg ccttccctct gaactcctac atgaacgcca ccaaccacgc catcgtgcag 1140
   acgctggtcc acttcatcaa cccggaaacg gtgcccaagc cctgctgtgc gcccacgcag 1200
   ctcaatgcca tctccgtcct ctacttcgat gacagctcca acgtcatcct gaagaaatac 1260
   gaagacatgg tggtcgaggc ctgtggctgc cac                              1293
<210> 32
   <211> 431
   <212> PRT
   <213> Artificial Sequence
<220>
   <223> cDNA encoding a hBMP-7 precursor derived variant peptide chain comprising the delta R299S, R314S, and R421E, N422D, and R426E mutations.
<400> 32
   Met His Val Arg Ser Leu Arg Ala Ala Ala Pro His Ser Phe Val Ala
   1 5 10 15
   Leu Trp Ala Pro Leu Phe Leu Leu Arg Ser Ala Leu Ala Asp Phe Ser
   20 25 30
   Leu Asp Asn Glu Val His Ser Ser Phe Ile His Arg Arg Leu Arg Ser
   35 40 45
   Gln Glu Arg Arg Glu Met Gln Arg Glu Ile Leu Ser Ile Leu Gly Leu
   50 55 60
   Pro His Arg Pro Arg Pro His Leu Gln Gly Lys His Asn Ser Ala Pro
   65 70 75 80
   Met Phe Met Leu Asp Leu Tyr Asn Ala Met Ala Val Glu Glu Gly Gly
   85 90 95
   Gly Pro Gly Gly Gln Gly Phe Ser Tyr Pro Tyr Lys Ala Val Phe Ser
   100 105 110
   Thr Gln Gly Pro Pro Leu Ala Ser Leu Gln Asp Ser His Phe Leu Thr
   115 120 125
   Asp Ala Asp Met Val Met Ser Phe Val Asn Leu Val Glu His Asp Lys
   130 135 140
   Glu Phe Phe His Pro Arg Tyr His His Arg Glu Phe Arg Phe Asp Leu
   145 150 155 160
   Ser Lys Ile Pro Glu Gly Glu Ala Val Thr Ala Ala Glu Phe Arg Ile
   165 170 175
   Tyr Lys Asp Tyr Ile Arg Glu Arg Phe Asp Asn Glu Thr Phe Arg Ile
   180 185 190
   Ser Val Tyr Gln Val Leu Gln Glu His Leu Gly Arg Glu Ser Asp Leu
   195 200 205
   Phe Leu Leu Asp Ser Arg Thr Leu Trp Ala Ser Glu Glu Gly Trp Leu
   210 215 220
   Val Phe Asp Ile Thr Ala Thr Ser Asn His Trp Val Val Asn Pro Arg
   225 230 235 240
   His Asn Leu Gly Leu Gln Leu Ser Val Glu Thr Leu Asp Gly Gln Ser
   245 250 255
   Ile Asn Pro Lys Leu Ala Gly Leu Ile Gly Arg His Gly Pro Gln Asn
   260 265 270
   Lys Gln Pro Phe Met Val Ala Phe Phe Lys Ala Thr Glu Val His Phe
   275 280 285
   Arg Ser Ile Arg Ser Thr Gly Ser Lys Gln Ser Ser Gln Asn Arg Ser
   290 295 300
   Lys Thr Pro Lys Asn Gln Glu Ala Leu Ser Met Ala Asn Val Ala Glu
   305 310 315 320
   Asn Ser Ser Ser Asp Gln Arg Gln Ala Cys Lys Lys His Glu Leu Tyr
   325 330 335
   Val Ser Phe Arg Asp Leu Gly Trp Gln Asp Trp Ile Ile Ala Pro Glu
   340 345 350
   Gly Tyr Ala Ala Tyr Tyr Cys Glu Gly Glu Cys Ala Phe Pro Leu Asn
   355 360 365
   Ser Tyr Met Asn Ala Thr Asn His Ala Ile Val Gln Thr Leu Val His
   370 375 380
   Phe Ile Asn Pro Glu Thr Val Pro Lys Pro Cys Cys Ala Pro Thr Gln
   385 390 395 400
   Leu Asn Ala Ile Ser Val Leu Tyr Phe Asp Asp Ser Ser Asn Val Ile
   405 410 415
   Leu Lys Lys Tyr Glu Asp Met Val Val Glu Ala Cys Gly Cys His
   420 425 430
<210> 33
   <211> 1293
   <212> DNA
   <213> Artificial Sequence
<220>
   <223> cDNA encoding a hBMP-7 precursor derived variant peptide chain comprising the delta R293C, R299S, and R314S mutations.
<400> 33
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccggtgca cggggagcaa acagagcagc 900
   cagaaccgct ccaagacgcc caagaaccag gaagccctga gcatggccaa cgtggcagag 960
   aacagcagca gcgaccagag gcaggcctgt aagaagcacg agctgtatgt cagcttccga 1020
   gacctgggct ggcaggactg gatcatcgcg cctgaaggct acgccgccta ctactgtgag 1080
   ggggagtgtg ccttccctct gaactcctac atgaacgcca ccaaccacgc catcgtgcag 1140
   acgctggtcc acttcatcaa cccggaaacg gtgcccaagc cctgctgtgc gcccacgcag 1200
   ctcaatgcca tctccgtcct ctacttcgat gacagctcca acgtcatcct gaagaaatac 1260
   agaaacatgg tggtccgggc ctgtggctgc cac                              1293
<210> 34
   <211> 431
   <212> PRT
   <213> Artificial Sequence
<220>
   <223> cDNA encoding a hBMP-7 precursor derived variant peptide chain comprising the delta R293C, R299S, and R314S mutations.
<400> 34
   Met His Val Arg Ser Leu Arg Ala Ala Ala Pro His Ser Phe Val Ala
    1               5                  10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Cys Thr Gly Ser Lys Gln Ser Ser Gln Asn Arg Ser
       290                 295                 300
   Lys Thr Pro Lys Asn Gln Glu Ala Leu Ser Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Val His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Leu Tyr Phe Asp Asp Ser Ser Asn Val Ile
                   405                 410                 415
   Leu Lys Lys Tyr Arg Asn Met Val Val Arg Ala Cys Gly Cys His
               420                 425                 430
<210> 35
   <211> 876
   <212> DNA
   <213> Artificial Sequence
<220>
   <223> cDNA encoding a hBMP-7 precursor derived variant peptide chain comprising the native hBMP-7 signal peptide and prodomain alone due to a deletion mutation that removed the hBMP-7 mature form domain.
<400> 35
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtcca cttccgcagc atccgg                          876
<210> 36
   <211> 292
   <212> PRT
   <213> Artificial Sequence
<220>
   <223> cDNA encoding a hBMP-7 precursor derived variant peptide chain comprising the native hBMP-7 signal peptide and prodomain alone due to a deletion mutation that removed the hBMP-7 mature form domain.
<400> 36
   Met His Val Arg Ser Leu Arg Ala Ala Ala Pro His Ser Phe Val Ala
   1               5                   10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg
       290
<210> 37
   <211> 495
   <212> DNA
   <213> Artificial Sequence
<220>
   <223> Homo sapiens derived cDNA encoding a hBMP-7 precursor derived variant peptide chain comprising the human growth hormone signal peptide instead of the nature hBMP-7 secretory signal peptide and the R299S and R314S utation in the hBMP-7 mature form domain.
<400> 37
   atggctacag gctcccggac gtccctgctc ctggcttttg gcctgctctg cctgccctgg 60
   cttcaagagg gatccgcctc cacggggagc aaacagagca gccagaaccg ctccaagacg 120
   cccaagaacc aggaagccct gagcatggcc aacgtggcag agaacagcag cagcgaccag 180
   aggcaggcct gtaagaagca cgagctgtat gtcagcttcc gagacctggg ctggcaggac 240
   tggatcatcg cgcctgaagg ctacgccgcc tactactgtg agggggagtg tgccttccct 300
   ctgaactcct acatgaacgc caccaaccac gccatcgtgc agacgctggt ccacttcatc 360
   aacccggaaa cggtgcccaa gccctgctgt gcgcccacgc agctcaatgc catctccgtc 420
   ctctacttcg atgacagctc caacgtcatc ctgaagaaat acagaaacat ggtggtccgg 480
   gcctgtggct gccac                                                  495
<210> 38
   <211> 165
   <212> PRT
   <213> Artificial Sequence
<220>
   <223> Homo sapiens derived cDNA encoding a hBMP-7 precursor derived variant peptide chain comprising the human growth hormone signal peptide instead of the nature hBMP-7 secretory signal peptide and the R299S and R314S utation in the hBMP-7 mature form domain.
<400> 38
   Met Ala Thr Gly Ser Arg Thr Ser Leu Leu Leu Ala Phe Gly Leu Leu
    1               5                  10                  15
   Cys Leu Pro Trp Leu Gln Glu Gly Ser Ala Ser Thr Gly Ser Lys Gln
               20                  25                  30
   Ser Ser Gln Asn Arg Ser Lys Thr Pro Lys Asn Gln Glu Ala Leu Ser
           35                  40                  45
   Met Ala Asn Val Ala Glu Asn Ser Ser Ser Asp Gln Arg Gln Ala Cys
       50                  55                  60
   Lys Lys His Glu Leu Tyr Val Ser Phe Arg Asp Leu Gly Trp Gln Asp
   65                  70                  75                  80
   Trp Ile Ile Ala Pro Glu Gly Tyr Ala Ala Tyr Tyr Cys Glu Gly Glu
                   85                  90                  95
   Cys Ala Phe Pro Leu Asn Ser Tyr Met Asn Ala Thr Asn His Ala Ile
               100                 105                 110
   Val Gln Thr Leu Val His Phe Ile Asn Pro Glu Thr Val Pro Lys Pro
           115                 120                 125
   Cys Cys Ala Pro Thr Gln Leu Asn Ala Ile Ser Val Leu Tyr Phe Asp
       130                 135                 140
   Asp Ser Ser Asn Val Ile Leu Lys Lys Tyr Arg Asn Met Val Val Arg
   145                 150                 155                 160
   Ala Cys Gly Cys His
                   165
<210> 39
   <211> 1263
   <212> DNA
   <213> Artificial Sequence
<220>
   <223> Homo sapiens derived cDNA encoding a hBMP-7 precursor derived variant peptide chain comprising the Homo sapiens BMP-2 (hBMP-2) secretory signal peptide instead of the nature hBMP-7 secretory signal peptide, the hBMP-2 prodomain instead of the nature hBMP-7 prodomain, and the R299S and R314S mutations in the hBMP-7 mature form domain.
<400> 39
   atggtggccg ggacccgctg tcttctagcg ttgctgcttc cccaggtcct cctgggcggc 60
   gcggctggcc tcgttccgga gctgggccgc aggaagttcg cggcggcgtc gtcgggccgc 120
   ccctcatccc agccctctga cgaggtcctg agcgagttcg agttgcggct gctcagcatg 180
   ttcggcctga aacagagacc cacccccagc agggacgccg tggtgccccc ctacatgcta 240
   gacctgtatc gcaggcactc aggtcagccg ggctcacccg ccccagacca ccggttggag 300
   agggcagcca gccgagccaa cactgtgcgc agcttccacc atgaagaatc tttggaagaa 360
   ctaccagaaa cgagtgggaa aacaacccgg agattcttct ttaatttaag ttctatcccc 420
   acggaggagt ttatcacctc agcagagctt caggttttcc gagaacagat gcaagatgct 480
   ttaggaaaca atagcagttt ccatcaccga attaatattt atgaaatcat aaaacctgca 540
   acagccaact cgaaattccc cgtgaccaga cttttggaca ccaggttggt gaatcagaat 600
   gcaagcaggt gggaaagttt tgatgtcacc cccgctgtga tgcggtggac tgcacaggga 660
   cacgccaacc atggattcgt ggtggaagtg gcccacttgg aggagaaaca aggtgtctcc 720
   aagagacatg ttaggataag caggtctttg caccaagatg aacacagctg gtcacagata 780
   aggccattgc tagtaacttt tggccatgat ggaaaagggc atcctctcca caaaagagaa 840
   aaaagatcta cggggagcaa acagagcagc cagaaccgct ccaagacgcc caagaaccag 900
   gaagccctga gcatggccaa cgtggcagag aacagcagca gcgaccagag gcaggcctgt 960
   aagaagcacg agctgtatgt cagcttccga gacctgggct ggcaggactg gatcatcgcg 1020
   cctgaaggct acgccgccta ctactgtgag ggggagtgtg ccttccctct gaactcctac 1080
   atgaacgcca ccaaccacgc catcgtgcag acgctggtcc acttcatcaa cccggaaacg 1140
   gtgcccaagc cctgctgtgc gcccacgcag ctcaatgcca tctccgtcct ctacttcgat 1200
   gacagctcca acgtcatcct gaagaaatac agaaacatgg tggtccgggc ctgtggctgc 1260
   cac                                                               1263
<210> 40
   <211> 421
   <212> PRT
   <213> Artificial Sequence
<220>
   <223> Homo sapiens derived cDNA encoding a hBMP-7 precursor derived variant peptide chain comprising the Homo sapiens BMP-2 (hBMP-2) secretory signal peptide instead of the nature hBMP-7 secretory signal peptide, the hBMP-2 prodomain instead of the nature hBMP-7 prodomain, and the R299S and R314S mutations in the hBMP-7 mature form domain.
<400> 40
   Met Val Ala Gly Thr Arg Cys Leu Leu Ala Leu Leu Leu Pro Gln Val
   1               5                   10                  15
   Leu Leu Gly Gly Ala Ala Gly Leu Val Pro Glu Leu Gly Arg Arg Lys
               20                  25                  30
   Phe Ala Ala Ala Ser Ser Gly Arg Pro Ser Ser Gln Pro Ser Asp Glu
           35                  40                  45
   Val Leu Ser Glu Phe Glu Leu Arg Leu Leu Ser Met Phe Gly Leu Lys
       50                  55                  60
   Gln Arg Pro Thr Pro Ser Arg Asp Ala Val Val Pro Pro Tyr Met Leu
   65                  70                  75                  80
   Asp Leu Tyr Arg Arg His Ser Gly Gln Pro Gly Ser Pro Ala Pro Asp
                   85                  90                  95
   His Arg Leu Glu Arg Ala Ala Ser Arg Ala Asn Thr Val Arg Ser Phe
               100                 105                 110
   His His Glu Glu Ser Leu Glu Glu Leu Pro Glu Thr Ser Gly Lys Thr
           115                 120                 125
   Thr Arg Arg Phe Phe Phe Asn Leu Ser Ser Ile Pro Thr Glu Glu Phe
       130                 135                 140
   Ile Thr Ser Ala Glu Leu Gln Val Phe Arg Glu Gln Met Gln Asp Ala
   145                 150                 155                 160
   Leu Gly Asn Asn Ser Ser Phe His His Arg Ile Asn Ile Tyr Glu Ile
                   165                 170                 175
   Ile Lys Pro Ala Thr Ala Asn Ser Lys Phe Pro Val Thr Arg Leu Leu
               180                 185                 190
   Asp Thr Arg Leu Val Asn Gln Asn Ala Ser Arg Trp Glu Ser Phe Asp
           195                 200                 205
   Val Thr Pro Ala Val Met Arg Trp Thr Ala Gln Gly His Ala Asn His
       210                 215                 220
   Gly Phe Val Val Glu Val Ala His Leu Glu Glu Lys Gln Gly Val Ser
   225                 230                 235                 240
   Lys Arg His Val Arg Ile Ser Arg Ser Leu His Gln Asp Glu His Ser
                   245                 250                 255
   Trp Ser Gln Ile Arg Pro Leu Leu Val Thr Phe Gly His Asp Gly Lys
               260                 265                 270
   Gly His Pro Leu His Lys Arg Glu Lys Arg Ser Thr Gly Ser Lys Gln
           275                 280                 285
   Ser Ser Gln Asn Arg Ser Lys Thr Pro Lys Asn Gln Glu Ala Leu Ser
       290                 295                 300
   Met Ala Asn Val Ala Glu Asn Ser Ser Ser Asp Gln Arg Gln Ala Cys
   305                 310                 315                 320
   Lys Lys His Glu Leu Tyr Val Ser Phe Arg Asp Leu Gly Trp Gln Asp
                   325                 330                 335
   Trp Ile Ile Ala Pro Glu Gly Tyr Ala Ala Tyr Tyr Cys Glu Gly Glu
               340                 345                 350
   Cys Ala Phe Pro Leu Asn Ser Tyr Met Asn Ala Thr Asn His Ala Ile
           355                 360                 365
   Val Gln Thr Leu Val His Phe Ile Asn Pro Glu Thr Val Pro Lys Pro
       370                 375                 380
   Cys Cys Ala Pro Thr Gln Leu Asn Ala Ile Ser Val Leu Tyr Phe Asp
   385                 390                 395                 400
   Asp Ser Ser Asn Val Ile Leu Lys Lys Tyr Arg Asn Met Val Val Arg
                   405                 410                 415
   Ala Cys Gly Cys His
               420
<210> 41
   <211> 1293
   <212> DNA
   <213> Artificial Sequence
<220>
   <223> hBMP-7 precursor derived variant peptide chain comprising the Homo sapiens BMP-2 (hBMP-2) secretory signal peptide instead of the nature hBMP-7 secretory signal peptide, the hBMP-2 prodomain instead of the nature hBMP-7 prodomain, and the R299S and R314S mutations in the hBMP-7 mature form domain. This hBMP-7 precursor derived variant peptide chain is encoded by a Homo sapiens derived cDNA.
<400> 41
   atgcacgtgc gctcactgcg agctgcggcg ccgcacagct tcgtggcgct ctgggcaccc 60
   ctgttcctgc tgcgctccgc cctggccgac ttcagcctgg acaacgaggt gcactcgagc 120
   ttcatccacc ggcgcctccg cagccaggag cggcgggaga tgcagcgcga gatcctctcc 180
   attttgggct tgccccaccg cccgcgcccg cacctccagg gcaagcacaa ctcggcaccc 240
   atgttcatgc tggacctgta caacgccatg gcggtggagg agggcggcgg gcccggcggc 300
   cagggcttct cctaccccta caaggccgtc ttcagtaccc agggcccccc tctggccagc 360
   ctgcaagata gccatttcct caccgacgcc gacatggtca tgagcttcgt caacctcgtg 420
   gaacatgaca aggaattctt ccacccacgc taccaccatc gagagttccg gtttgatctt 480
   tccaagatcc cagaagggga agctgtcacg gcagccgaat tccggatcta caaggactac 540
   atccgggaac gcttcgacaa tgagacgttc cggatcagcg tttatcaggt gctccaggag 600
   cacttgggca gggaatcgga tctcttcctg ctcgacagcc gtaccctctg ggcctcggag 660
   gagggctggc tggtgtttga catcacagcc accagcaacc actgggtggt caatccgcgg 720
   cacaacctgg gcctgcagct ctcggtggag acgctggatg ggcagagcat caaccccaag 780
   ttggcgggcc tgattgggcg gcacgggccc cagaacaagc agcccttcat ggtggctttc 840
   ttcaaggcca cggaggtccg cttccgcagc atccggtcca cggggagcaa acagagcagc 900
   cagaaccgct ccaagacgcc caagaaccag gaagccctga gcatggccaa cgtggcagag 960
   aacagcagca gcgaccagag gcaggcctgt aagaagcacg agctgtatgt cagcttccga 1020
   gacctgggct ggcaggactg gatcatcgcg cctgaaggct acgccgccta ctactgtgag 1080
   ggggagtgtg ccttccctct gaactcctac atgaacgcca ccaaccacgc catcgtgcag 1140
   acgctggtcc acttcatcaa cccggaaacg gtgcccaagc cctgctgtgc gcccacgcag 1200
   ctcaatgcca tctccgtcct ctacttcgat gacagctcca acgtcatcct gaagaaatac 1260
   gaagacatgg tggtcgaggc ctgtggctgc cac 1293
<210> 42
   <211> 431
   <212> PRT
   <213> Artificial Sequence
<220>
   <223> hBMP-7 precursor derived variant peptide chain comprising the H258R, R299S, R314S, R421E, N422D, and R426E mutations and encoded by a Homo sapiens derived cDNA.
<400> 42
   Met His Val Arg Ser Leu Arg Ala Ala Ala Pro His Ser Phe Val Ala
   1               5                   10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Gly Gly
                   85                  90                  95
   Gly Pro Gly Gly Gln Gly Phe Ser Tyr Pro Tyr Lys Ala Val Phe Ser
               100                 105                 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val Arg Phe
           275                 280                 285
   Arg Ser Ile Arg Ser Thr Gly Ser Lys Gln Ser Ser Gln Asn Arg Ser
       290                 295                 300
   Lys Thr Pro Lys Asn Gln Glu Ala Leu Ser Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Val His
       370                 375                 380
   Phe Ile Asn Pro Glu Thr Val Pro Lys Pro Cys Cys Ala Pro Thr Gln
   385                 390                 395                 400
   Leu Asn Ala Ile Ser Val Leu Tyr Phe Asp Asp Ser Ser Asn Val Ile
                   405                 410                 415
   Leu Lys Lys Tyr Glu Asp Met Val Val Glu Ala Cys Gly Cys His
               420                 425                 430